# EUROPEAN PATENT APPLICATION

(11) **EP 4 756 029 A1**
(43) Date of publication of application: **10.06.2026**
(21) Application number: 24851142.0
(22) Date of filing: 09.08.2024
(51) Int. Cl.: C12N 15/117, A61K 45/00, A61P 35/00

(54) **TOLL-LIKE RECEPTOR AGONISTS AND USE THEREOF**

(30) Priority: 10.08.2023 CN 202311008396; 20.03.2024 CN 202410324833; 09.05.2024 CN 202410567565
(71) Applicant: Haichang Biotech Co., Ltd., Zhejiang 310018 (CN)
(72) Inventor: BAI, Jun, Hangzhou, Zhejiang 310018 (CN); SU, Fei, Hangzhou, Zhejiang 310018 (CN); LIN, Jialiang, Hangzhou, Zhejiang 310018 (CN); LEE, Robert J., Hangzhou, Zhejiang 310018 (CN); YANG, Yongsheng, Hangzhou, Zhejiang 310018 (CN); ZHAO, Xiaobin, Hangzhou, Zhejiang 310018 (CN)
(74) Representative: Dobrzanski, Jan Pawel
(86) International application number: PCT/CN2024/111218
(87) International publication number: WO 2025/031498

(57) **Abstract**

Disclosed is a Toll-like receptor agonist, which is a Class C CpG-ODN that can effectively activate TLR9. Its nucleotide sequence comprises any one of the modified or unmodified nucleotide sequences set forth in SEQ ID NOs: 1-4. The Toll-like receptor agonist has a significant effect in inhibiting tumor growth. The lipid-encapsulated Toll-like receptor agonist provided can inhibit tumor growth, prevent tumor recurrence, and improve immune activation efficiency alone or in combination with an additional therapeutic agent, and exhibits improved performance compared to free Toll-like receptor agonists.

## Description

### Cross-Reference to Related Applications

The present application claims priority to CN202311008396.X filed on August 10, 2023, CN202410324833.7 filed on March 20, 2024, and CN202410567565.1 filed on May 9, 2024. The prior applications are considered part of the disclosure of the present application and are incorporated herein by reference in their entirety.

### Technical Field

The present disclosure relates to the field of biomedicine, and specifically to Toll-like receptor agonists.

### Background

Toll-like receptors (TLRs) are highly conserved pattern recognition receptors found in various cell types and play a key role in innate immune cell surveillance of microbial pathogens. Ten TLRs have been identified in humans, expressed on T cells, B cells, APCs, and various non-immune cells including epithelial and endothelial cells. TLRs are localized in two distinct cellular compartments: the cell membrane and intracellular endosomes. TLR1, 2, 5, and 6 reside on the plasma membrane, TLR3, 7, 8, and 9 are expressed in intracellular endosomes, and TLR4 is present in both compartments. TLRs are expressed on both immune cells and tumor cells within the tumor microenvironment, and their activation can inhibit Treg cell function and enhance the survival, proliferation, and cytokine release of CD8⁺ T cells.

To date, numerous studies have explored using TLR agonists for cancer treatment. Since TLR9 activation can induce the activation of dendritic cells, T cells, and B cells, and promote T cell migration to the tumor microenvironment by inducing interferon-alpha secretion, TLR9 agonists show great potential in cancer therapy. TLR9 agonists primarily consist of bacterial unmethylated CpG DNA, and synthetic CpG oligodeoxynucleotides (CpG-ODNs) have also been proven to effectively activate TLR9. CpG-ODNs are generally categorized into three classes: A, B, and C. Class A CpG-ODNs have a palindromic sequence containing CpG dinucleotides at their core, flanked by poly-G tails, with a phosphodiester backbone that is partially phosphorothioate-modified. They form higher-order structures via the palindrome and poly-G tails, enabling the activation of plasmacytoid dendritic cells to induce large amounts of Type I interferons, but have weak activity on B cells. Class B CpG-ODNs are fully phosphorothioate-modified linear CpG-ODNs that have strong immunostimulatory activity on B cells but cannot activate plasmacytoid dendritic cells. Class C CpG-ODNs are fully phosphorothioate-modified CpG-ODNs capable of forming dimers via palindromic sequences. They combine the activities of both Class A and Class B CpG-ODNs, activating both plasmacytoid dendritic cells and B cells. CpG-ODNs are used as vaccine adjuvants to enhance immune responses, and some products have been approved for marketing, such as the HEPLISAV-B hepatitis B vaccine. Besides vaccines, TLR9 agonists can also be used for treating inflammation-related diseases and infectious diseases, and these applications are currently in clinical stages.

Despite the significant potential of TLR9 agonists in cancer therapy, developing them into anti-tumor drugs faces the following challenges: 1) Systemic administration of TLR9 agonists can cause systemic inflammation with significant side effects, and only a small fraction of the drug reaches the tumor microenvironment, resulting in poor efficacy; 2) Some researchers use intratumoral injection to reduce the administered dose to some extent, but the drug diffuses rapidly, with very little remaining within the tumor; 3) TLR9 is an intracellular receptor, and only a small amount of naked CpG-ODN can enter cells to exert its effects.

Current optimization of TLR9 agonists primarily focuses on preventing rapid degradation of the oligonucleotide and enhancing endocytosis. Mologen AG's product Lefitolimod extended the *in vivo* half-life of the oligonucleotide by modifying the linear oligonucleotide into a dumbbell-shaped structure. However, Phase II and III clinical trials of monotherapy for advanced colorectal cancer after first-line therapy and small cell lung cancer after first-line therapy showed unsatisfactory results, with efficacy not being apparent; hence, there are currently no ongoing cancer clinical trials. Checkmate's intratumoral injection product, CMP-001, is a CpG-A type TLR9 agonist encapsulated in virus-like particles to prevent degradation by nucleases, significantly extending its half-life. However, using virus-like particles for oligonucleotide delivery involves complex manufacturing processes and high production costs.

### Summary

The present disclosure provides Toll-like receptor agonists with tumor-suppressing effects, and further provides lipid-encapsulated Toll-like receptor agonists or multi-active ingredients of Toll-like receptor agonists in combination with additional therapeutic agents. The Toll-like receptor agonists encapsulated by lipid component or multi-active ingredients of Toll-like receptor agonists in combination with additional therapeutic agents provided herein exhibit significant broad-spectrum tumor inhibition, can prevent tumor recurrence, and prevent tumor metastasis. Compared to free Toll-like receptor agonists, the Toll-like receptor agonists encapsulated by lipid component or multi-active ingredients of Toll-like receptor agonists in combination with additional therapeutic agents provided herein show prolonged intratumoral retention, possess immunostimulatory effects, can upregulate cytokine expression, exert broad-spectrum tumor inhibition upon systemic administration, have effects in preventing tumor recurrence and metastasis, effectively extend the survival period of test animals, and demonstrate good safety and tolerability. The Toll-like receptor agonists encapsulated by lipid component or multi-active ingredients of Toll-like receptor agonists in combination with additional therapeutic agents provided herein have fewer side effects compared to free Toll-like receptor agonists and show positive efficacy expectations in combating human tumors.

In a first aspect, the present disclosure provides a Toll-like receptor agonist characterized by comprising any one of the following (a)-(c): (a) a modified or unmodified nucleotide set forth in any one of SEQ ID NOs: 1-4; (b) a functional variant of a modified or unmodified nucleotide set forth in any one of SEQ ID NOs: 1-4; (c) a pharmaceutically acceptable salt of the nucleotide of (a) or (b); wherein the nucleotide is entirely or partially composed of ribonucleotides or entirely or partially composed of deoxyribonucleotides.

In a second aspect, the present disclosure provides an active ingredient encapsulated by a lipid component, characterized in that the active ingredient comprises the Toll-like receptor agonist provided in the first aspect of the present disclosure. In some embodiments, the lipid component comprises DOTAP, DODMA, DSPC, cholesterol, and DMG-PEG.

In a third aspect, the present disclosure provides a method for preparing an active ingredient encapsulated by a lipid component, characterized by comprising the following steps (a)-(f):
(a) preparing the Toll-like receptor agonist provided in the first aspect of the present disclosure as an acidic solution;
(b) preparing each component of the lipid components as a lipid ethanol solution;
(c) mixing and reacting the solutions prepared in steps (a) and (b) to form an initial lipid nanoparticle solution;
(d) optionally repeating steps (a)-(c) to obtain more initial lipid nanoparticle solutions;
(e) removing ethanol from the initial lipid nanoparticle solution, adjusting the active ingredient concentration and pH to obtain a lipid nanoparticle suspension;
(f) optionally mixing an additional therapeutic agent with the lipid nanoparticle suspension to prepare a multi-active-ingredient liposome.

In a fourth aspect, the present disclosure provides a composition characterized by comprising the active ingredient encapsulated by a lipid component provided in the second aspect of the present disclosure and an additional therapeutic agent.

In a fifth aspect, the present disclosure provides a delivery system characterized by comprising an active ingredient and a lipid component, wherein the active ingredient comprises the Toll-like receptor agonist provided in the first aspect of the present disclosure.

In a sixth aspect, the present disclosure provides a pharmaceutical composition characterized by comprising the Toll-like receptor agonist provided in the first aspect of the present disclosure, the active ingredient encapsulated by a lipid component provided in the second aspect, the composition provided in the fourth aspect, or the delivery system provided in the fifth aspect, and a pharmaceutically acceptable carrier or excipient.

In a seventh aspect, the present disclosure provides a vaccine adjuvant or a vaccine characterized by comprising the Toll-like receptor agonist provided in the first aspect of the present disclosure, the active ingredient encapsulated by a lipid component provided in the second aspect, the composition provided in the fourth aspect, the delivery system provided in the fifth aspect, or the pharmaceutical composition provided in the sixth aspect.

In an eighth aspect, the present disclosure provides the use of the Toll-like receptor agonist according to the first aspect, the active ingredient encapsulated by a lipid component according to the second aspect, the composition according to the fourth aspect, the delivery system according to the fifth aspect, or the pharmaceutical composition according to the sixth aspect in any one of the following (a)-(j):
(a) use in the manufacture of a medicament for preventing tumorigenesis;
(b) use in the manufacture of a medicament for treating a tumor;
(c) use in the manufacture of a medicament for inhibiting tumor growth;
(d) use in the manufacture of a medicament for preventing tumor cell metastasis;
(e) use in the manufacture of a medicament for preventing tumor recurrence;
(f) use in the manufacture of a vaccine;
(g) use in the manufacture of a medicament for treating or preventing an inflammation-related disease or an infectious disease;
(h) use in the manufacture of a medicament for immune activation;
(i) use in the manufacture of a medicament for upregulating cytokine expression;
(j) use for upregulating cytokine expression for non-therapeutic purpose.

In a ninth aspect, the present disclosure provides a method of any one of the following (a)-(h):
(a) a method for preventing tumorigenesis;
(b) a method for treating a tumor;
(c) a method for inhibiting tumor growth;
(d) a method for preventing tumor cell metastasis;
(e) a method for preventing tumor recurrence;
(f) a method for treating or preventing an inflammation-related disease or an infectious disease;
(g) a method for immune activation;
(h) a method for upregulating cytokine expression;
characterized by comprising administering a therapeutically effective amount of the Toll-like receptor agonist according to the first aspect of the present disclosure, the active ingredient encapsulated by a lipid component according to the second aspect, the composition according to the fourth aspect, the delivery system according to the fifth aspect, or the pharmaceutical composition according to the sixth aspect to a subject in need thereof.

In a tenth aspect, the present disclosure provides a method for preparing a delivery system, characterized by comprising the following steps (a)-(d):
(a) preparing a Toll-like receptor agonist buffer solution using a buffer and the Toll-like receptor agonist according to the first aspect of the present disclosure;
(b) preparing a lipid ethanol solution using anhydrous ethanol and the lipid component;
(c) mixing the lipid ethanol solution and the Toll-like receptor agonist buffer solution at a mass ratio of lipid component to Toll-like receptor agonist of (8-12): 1;
(d) removing ethanol, diluting, and adjusting the pH to 6.8-7.2.

### Brief Description of the Figures

Fig. 1 shows the tumor volume change curve over time during intratumoral administration in MC38 tumor-bearing mice when administered saline or different CpG-ODNs, namely T0009-1, T0009-2, T0009-3, and T0009-4, in Example 1.
Fig. 2 shows the tumor volume change curve over time during intratumoral administration in MC38 tumor-bearing mice when administered saline or Qtolimod prepared from different CpG-ODNs (T0009-1 Qtolimod, T0009-2 Qtolimod, T0009-3 Qtolimod, T0009-4 Qtolimod) in Example 8. "i.t." represents intratumoral injection, and "q3d*5" represents once every 3 days for a total of 5 times.
Fig. 3 shows the body weight change rate (%) curve over time during intratumoral administration in MC38 tumor-bearing mice when administered saline or Qtolimod prepared from different CpG-ODNs in Example 8. "i.t." represents intratumoral, and "q3d*5" represents once every 3 days for a total of 5 times.
Fig. 4 shows the tumor volume change curve over time during administration in B16F10 tumor-bearing mice when administered saline or Qtolimod prepared from different CpG-ODNs in Example 9.
Fig. 5 shows the body weight change rate (%) curve over time during administration in B16F10 tumor-bearing mice when administered saline or Qtolimod prepared from different CpG-ODNs in Example 9.
Fig. 6 shows the survival rate curve over time during intratumoral administration in B16F10 tumor-bearing mice when administered saline or Qtolimod prepared from different CpG-ODNs in Example 9.
Fig. 7 shows the tumor volume change curve over time during administration in MC38 tumor-bearing mice when administered saline, T0009-1 Qtolimod, Tritolimod, or Doxotolimod in Example 10 (the first tumor measurement time was the day before the first administration, Day -1).
Fig. 8 shows the body weight change rate (%) curve over time during administration in MC38 tumor-bearing mice when administered saline, T0009-1 Qtolimod, Tritolimod, or Doxotolimod in Example 10.
Fig. 9 shows the tumor volume change curve over time during tail vein administration in B16F10 tumor-bearing mice when administered Tritolimod or saline in Example 11.
Fig. 10 shows the survival rate curve over time during tail vein administration in B16F10 tumor-bearing mice when administered Tritolimod or saline in Example 11.
Fig. 11 shows the body weight change rate (%) curve over time during intratumoral administration in MC38 tumor-bearing mice when administered saline, lipid solution (QTsome group), 0.5 mg/kg, 1 mg/kg, 1.5 mg/kg, or 2 mg/kg T0009-1 Qtolimod in Example 12 (the first tumor measurement time was the day before the first administration, Day -1).
Fig. 12 shows the tumor volume change curve over time during intratumoral administration in MC38 tumor-bearing mice when administered saline, lipid solution (QTsome group), 0.5 mg/kg, 1 mg/kg, 1.5 mg/kg, or 2 mg/kg T0009-1 Qtolimod in Example 12 (the first tumor measurement time was the day before the first administration, Day -1).
Fig. 13A shows the tumor volume change curves over time after re-establishing a control group on Day 36, subcutaneously implanting MC38 cells into the left shoulder of mice in the control group and each Qtolimod group, in tumor-bearing mice (no further administration to any group).
Fig. 13B shows the left shoulder tumor volume in tumor-bearing mice at the experimental endpoint after re-establishing a control group on Day 36, subcutaneously implanting MC38 cells into the left shoulder of mice in the control group and each Qtolimod group (no further administration to any group).
Fig. 14A shows *in vivo* drug imaging at different time points after intratumoral administration of T0009-1 Qtolimod and free T0009-1 in MC38 tumor-bearing mice in Example 13. Free T0009-1 was administered to the left mouse, and T0009-1 Qtolimod was administered to the right mouse. The colored areas on the mice correspond to the main drug distribution areas, with colors tending towards yellow indicating high fluorescence intensity, towards red indicating medium fluorescence intensity, and towards black indicating low fluorescence intensity.
Fig. 14B shows histogram of drug imaging fluorescence intensity at different time points after intratumoral administration of T0009-1 Qtolimod and free T0009-1 in MC38 tumor-bearing mice in Example 13.
Fig. 15A shows CD11b immunohistochemistry images (left) and CD11b-positive area ratio (right) of tumor sections from mice administered saline or T0009-1 Qtolimod in Example 14. Brown color in the immunohistochemistry images indicates CD11b positivity.
Fig. 15B shows CD8 immunohistochemistry images (left) and the percentage of CD8-positive T cells relative to total tumor cells (right) of tumor sections from mice administered saline or T0009-1 Qtolimod in Example 14.
Fig. 15C shows F4/80 immunohistochemistry images (left) and F4/80-positive area ratio (right) of tumor sections from tumor-bearing mice administered saline or T0009-1 Qtolimod in Example 14. Brown color in the immunohistochemistry images indicates F4/80 positivity.
Fig. 16A shows concentrations of IL-12, IL-10, and IFN-γ in serum samples and tumor samples from tumor-bearing mice after administration of saline, lipid solution (QTsome group), or T0009-1 Qtolimod in Example 14.
Fig. 16B shows concentrations of IL-1B, IL-6, and TNF-α in serum samples and tumor samples from tumor-bearing mice after administration of saline, lipid solution (QTsome group), or T0009-1 Qtolimod in Example 14.
Fig. 17 shows body weight change curves over time in female and male mice after subcutaneous administration of saline (vehicle control group), lipid solution (QTsome group), free T0009-1 (API group), or T0009-1 Qtolimod (Qtolimod group) in Example 15.
Fig. 18 shows survival curve of animals in each group during the administration period in Example 16.
Fig. 19 shows body weight change trends of animals in each group during the administration period in Example 16.
Fig. 20 shows subcutaneous tumor volume change trends of animals in each group during the administration period in Example 16.

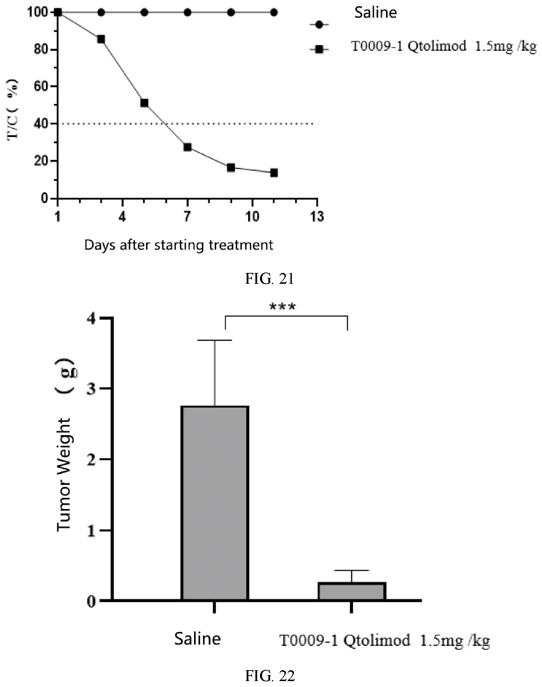

Fig. 21 shows changes in the relative tumor proliferation rate T/C values of animals in each group during the administration period in Example 16.
Fig. 22 shows subcutaneous tumor weights of animals in each group in Example 16.
Fig. 23 shows the distribution of metastatic tumor nodules in various tissues of animals in Example 16.
Fig. 24 shows the number of metastatic tumor nodules in lung tissue of animals of each group in Example 16.
Fig. 25 shows the total number of metastatic tumor nodules in animals of each group in Example 16.
Fig. 26 shows subcutaneous tumor volume change trends of animals in each group during the administration period in Example 17.
Fig. 27 shows body weight change trends of animals in each group during the administration period in Example 17.
Fig. 28A shows subcutaneous tumor volume change trends of animals in each EMT6 group during the administration period in Example 18.
Fig. 28B shows body weight change trends of animals in each EMT6 group during the administration period in Example 18.
Fig. 28C shows subcutaneous tumor volume change trends of animals in each EMT6-HPV18 group during the administration period in Example 18.
Fig. 28D shows body weight change trends of animals in each EMT6-HPV18 group during the administration period in Example 18.
Fig. 29A shows subcutaneous tumor volume change trends of animals in each K7M2 group during the administration period in Example 19.
Fig. 29B shows body weight change trends of animals in each K7M2 group during the administration period in Example 19.
Fig. 29C shows survival of animals in each group during the observation period after administration in Example 19.
Fig. 30 shows immune cell detection in tumor tissues at different time points after different number of administrations in tumor-bearing mice via intratumoral injection in Example 20. There were 3 mouse samples per time point, and each dot in the figure represents one sample.
Fig. 31 shows immune cell detection in spleen tissues at different time points after different number of administrations in tumor-bearing mice via intratumoral injection in Example 20. There were 3 mouse samples per time point, and each dot in the figure represents one sample.
Fig. 32 shows proteomic sequencing data analysis after *in vitro* stimulation of mouse and human PBMC cells in Example 21.
Fig. 33 shows anatomical pathology images after administration in a mouse orthotopic bladder cancer model in Example 22.

### Detailed Description of the Invention

Unless otherwise specified, the terms used herein have the usual meanings understood by those skilled in the relevant art. Those skilled in the art can vary these terms based on the desired properties and effects sought through this application. Each numerical parameter should be interpreted in view of the number of significant digits and ordinary rounding methods or as understood by those skilled in the art. In general, the nomenclature and the experimental procedures of organic chemistry, medicinal chemistry, and biology described herein are well-known and commonly used in the field. Unless otherwise defined, all technical and scientific terms used herein generally have the same meaning as commonly understood by one of ordinary skill in the art to which this application belongs. In case of multiple definitions for a term used herein, the definition in this section applies unless otherwise stated.

Unless otherwise indicated, all numbers expressing quantities, concentrations, proportions, weights, particle sizes, percentages, technical effects, etc., used in the specification and claims are to be understood as being modified in all instances by the term "about" or "approximately". Accordingly, unless indicated to the contrary, the numerical parameters set forth in the following specification and appended claims are approximations. "About" or "approximately" can be understood as a range of plus or minus 10%, 20%, 30%, 40%, or 50% of the indicated value.

As used herein, the expression "A and/or B" includes three cases: (1) A; (2) B; and (3) A and B. The expression "A, B and/or C" includes seven cases: (1) A; (2) B; (3) C; (4) A and B; (5) A and C; (6) B and C; and (7) A, B, and C. The meanings of similar expressions can be deduced accordingly.

As used herein, the terms "comprise", "include", and "contain" mean that in addition to the listed elements, other elements are not excluded.

### Definitions

As used herein, the term "pharmaceutically acceptable salt" includes acid addition salts and base addition salts. Suitable acid addition salts are formed from acids which form non-toxic salts. Examples include, but are not limited to: acetate, adipate, aspartate, benzoate, besylate, bicarbonate/carbonate, bisulfate/sulfate, borate, camphorsulfonate, citrate, cyclamate, edisylate, formate, fumarate, glucoheptonate, gluconate, glucuronate, hexafluorophosphate, 2-(4-hydroxybenzyl)benzoate, hydrochloride/chloride, hydrobromide/bromide, hydroiodide/iodide, 2-hydroxyethanesulfonate, lactate, malate, maleate, malonate, mesylate, methylsulfate, naphthalate, 2-naphthalenesulfonate, nicotinate, nitrate, orotate, oxalate, palmitate, phosphate/phosphate hydrogen/phosphate dihydrogen, pyroglutamate, saccharate, stearate, salicylate, tannate, tartrate, tosylate, and trifluoroacetate. Suitable base addition salts are formed from bases which form non-toxic salts. Examples include, but are not limited to: aluminum, arginine, calcium, choline, diethylamine, diethanolamine, glycine, lysine, magnesium, meglumine, ethanolamine, potassium, sodium, tromethamine, and zinc salts. Hemisalts of acids and bases may also be formed, e.g., hemisulfate and hemicalcium salts. For a review of suitable salts, see Handbook of Pharmaceutical Salts: Properties, Selection and Use by Stahl and Wermuth (Wiley-VCH, 2002).

As used herein, the term "functional variant" refers to a Toll-like receptor agonist having substantial or significant sequence identity or similarity to a parent Toll-like receptor agonist, CpG ODN, wherein the functional variant retains at least 40%, 50%, 60%, 70%, 80%, 90%, or 100% of the biological activity of the parent Toll-like receptor agonist.

### Toll-Like Receptor Agonist

The present disclosure provides a Toll-like receptor agonist characterized by comprising any one of the following (a)-(c): (a) a modified or unmodified nucleotide set forth in any one of SEQ ID NOs: 1-4; (b) a functional variant of a modified or unmodified nucleotide set forth in any one of SEQ ID NOs: 1-4; (c) a pharmaceutically acceptable salt of the nucleotide of (a) or (b); wherein the nucleotide is entirely or partially composed of ribonucleotides or entirely or partially composed of deoxyribonucleotides.

In some embodiments, the modification comprises one or more of the following: replacing the P=O bond between all or part of adjacent two nucleotides in the sequence with a P=S bond; replacing all or part of the deoxyribonucleotides in the sequence with ribonucleotides, or replacing all or part of the ribonucleotides in the sequence with deoxyribonucleotides; replacing the 2'-OH group of ribonucleotides or deoxyribonucleotides with methoxy, methoxyethyl, or halogen; bridging the 2nd and 4th carbon atoms of the nucleotide to form locked nucleic acid or (S)-cEt-BNA. In some embodiments, the halogen is selected from fluorine, chlorine, bromine, and iodine.

### Lipid-Encapsulated Active Ingredient

The present disclosure provides an active ingredient encapsulated by a lipid component, characterized in that the active ingredient comprises the Toll-like receptor agonist provided in the present disclosure.

In some embodiments, the mass ratio of the lipid component to the Toll-like receptor agonist provided in the present disclosure is (8-12):1, preferably 10:1.

In some embodiments, the lipid component comprises one or more of the following: cationic polymers, lipid nanoparticles (LNP), cationic liposomes, QTsome, lipopolyplexes, microparticles, microspheres, and nanoemulsions. QTsome is a lipid nanoparticle containing both quaternary and tertiary cationic phospholipids. In some embodiments, the present disclosure uses QTsome lipid nanotechnology to encapsulate CpG-ODNs. By introducing a combination of cationic lipids ionizable under specific conditions and utilizing the pH sensitivity of liposomes to the external environment, it maximizes oligonucleotide loading and facilitates intracellular delivery of CpG-ODNs.

In some embodiments, the lipid component comprises one or more of cationic lipids, ionizable lipids, neutral lipids, and PEGylated lipids.

Cationic lipids include, but are not limited to, DOTAP(CAS:132172-61-3, (2,3-Dioleoyloxy-propyl)-trimethylammonium-chloride), DOTMA (CAS No. 104872-42-6), DDBA, DMRIE, DOTIM, SAINT, DC-Chol (DC cholesterol, 3β-[N-(N',N'-dimethylaminoethane)-carbamoyl]cholesterol hydrochloride), BGTC, CTAP, DOSPA, DORIE, DODAB, DOIC, DMEPC, DOGS, DIMRI, DC-6-14, CLIP 1, DORIE, DOSPA, CLIP6, CLIP9, as well as those disclosed in U.S. Pat. No. 5,049,386, International Publications WO91/16024, WO97/019675, WO2005/121348, WO2009/086558, and WO2011/13636.

Ionizable lipids include, but are not limited to, tertiary amines and their derivatives, pyrrolidines and their derivatives, piperazines and their derivatives, piperidines and their derivatives, such as DODMA (1,2-dioleyloxy-3-dimethylaminopropane, CAS: 104162-47-2), ALC-0159, A066 (Z016) (1-(2,3-bis(((9Z,12Z)-octadeca-9,12-dien-1-yl)oxy)propyl)pyrrolidine), L-319 (CAS: 1351586-50-9), DODAP, C12-200, 5A2-SC8, 306Oi10, Moderna Lipid 5, Acuitas A9, ALC-0315, SM-102 (also known as HUO, Moderna Lipid H; 1-octylnonyl 5 8-[(2-hydroxyethyl)[6-oxo-6-(undecyloxy)hexyl]amino]-octanoate), DLin-MC3-DMA((6Z,9Z,28Z,31Z)-heptatriacont-6,9,28,31-tetraene-19-yl 4-(dimethylamino)butanoate), DLin-K-DMA, DLin-KC2-DMA, DLin-KC3-DMA, DLin-KC4-DMA, DLinDMA, other DLinDMA types, other DLin-K-DMA types, and lipid-like 5A2-SC8.

In some embodiments, neutral lipids include, but are not limited to, 1,2-dipalmitoyl-sn-glycero-3-phosphocholine (DPPC), dioleoyl-L-α-phosphatidylethanolamine (DOPE, CAS: 4004-05-1), 1,2-di-(9Z-octadecenoyl)-sn-glycero-3-phosphocholine (DOPC, CAS: 4235-95-4), 1-hexadecyl-2-(cis-9-octadecenoyl)-sn-glycero-3-phosphocholine (POPC), egg phosphatidylcholine (EPC), distearoylphosphatidylcholine (DSPC, CAS: 816-94-4), and cholesterol. In some embodiments, neutral lipids include neutral phospholipids, including but not limited to phosphatidylcholines and/or phosphatidylethanolamines, such as distearoylphosphatidylcholine (DSPC), egg lecithin (EPC), soybean lecithin, hydrogenated soybean lecithin (HSPC), dioleoylphosphatidylethanolamine (DOPE), dilauroylphosphatidylcholine (DLPC), dimyristoylphosphatidylcholine (DMPC), dipalmitoylphosphatidylcholine (DPPC), 1,2-dioleoylphosphatidylcholine (DOPC), diarachidoylphosphatidylcholine (DAPC), dimyristoylphosphatidylethanolamine (DMPE), dilauroylphosphatidylethanolamine (DLPE), distearoylphosphatidylethanolamine (DSPE), dipalmitoylphosphatidylethanolamine (DPPE), 1-palmitoyl-2-oleoyl-phosphatidylethanolamine (POPE), and 1-hexadecyl-2-(cis-9-octadecenoyl)-sn-glycero-3-phosphocholine (POPC).

In some embodiments, PEGylated lipids include, but are not limited to, PEG-didodecylacetamide, PEG-myristoyldiglyceride, PEG-diacylglycerol, PEG-dialkyloxypropyl, PEG-phospholipid, PEG-ceramide, DMG-PEG, PEG-DSPE, DSG-PEG. In some embodiments, the PEGylated lipid can be at least one selected from the group consisting of DSG-PEG and DMG-PEG.

In some embodiments, the lipid component comprises DOTAP, DODMA, DSPC, cholesterol, and DMG-PEG. In some preferred embodiments, the lipid component comprises DOTAP, DODMA, DSPC, cholesterol, and DMG-PEG, and the molar ratio of DOTAP:DODMA:DSPC:cholesterol:DMG-PEG is (1-30):(5-50):(5-40):(15-60):(0-10). In some more preferred embodiments, the lipid component comprises DOTAP, DODMA, DSPC, cholesterol, and DMG-PEG, and the molar ratio of DOTAP: DODMA: D SPC: cholesterol: DMG-PEG is 15:25:20:38.5:1.5.

In some embodiments, the active ingredient encapsulated by a lipid component comprises the Toll-like receptor agonist provided in the present disclosure and an additional therapeutic agent. In some preferred embodiments, the molar ratio of the additional therapeutic agent to the Toll-like receptor agonist provided in the present disclosure is (0.1-5):1, preferably 1:2.

In some embodiments, the additional therapeutic agent is one or more agents selected from the group consisting of antibodies, chemotherapeutic drugs, and small molecule drugs. In some embodiments, the additional therapeutic agent is one or more agents selected from the group consisting of polyinosinic:polycytidylic acid (Poly(I:C)), STING agonists, imiquimod, resiquimod, gardiquimod, sorafenib, sunitinib, erlotinib, doxorubicin, paclitaxel, gemcitabine, and radiotherapy. In some preferred embodiments, the additional therapeutic agent is one or both of resiquimod and doxorubicin.

In some embodiments, antibodies include, but are not limited to, antibodies with anti-tumor effects. Antibodies with anti-tumor effects include, but are not limited to: antibodies targeting PD-1, such as nivolumab, pembrolizumab, cemiplimab, toripalimab, sintilimab, camrelizumab, etc.; antibodies targeting PD-L1, such as atezolizumab, avelumab, and durvalumab; antibodies targeting CTLA-4, such as ipilimumab, tremelimumab, etc. In some embodiments, the combination of the Toll-like receptor agonist, CpG-ODNs, provided in the present disclosure with antibody drugs having anti-tumor effects, such as antibodies targeting PD-1 or PD-L1, can effectively enhance the responsiveness to immune checkpoint inhibitors or overcome resistance to immune checkpoint inhibitors, which is beneficial for further expanding the patient population for PD-1 or PD-L1 inhibitors, improving efficacy, and broadening indications.

In some embodiments, antibodies include, but are not limited to, anti-tumor-associated antigen (TAA) antibodies. TAAs include, but are not limited to, GPC, CEA, immature laminin receptor, TAG-72, HPV E6, HPV E7, EGFR, Ep-CAM, EphA3, Her2, Her3, ROR2, PSMA, STEAP1, FGFR2, TROP2, B7-H3, B7-H4, B7-H6, FOLR1, BAGE family, CAGE family, GAGE family, MAGE family, SAGE family, XAGE family, SSX-2, fibronectin, MART-2, PDL-1, VEGFR, CLAUDIN, etc.

In some embodiments, chemotherapeutic drugs include, but are not limited to, doxorubicin, paclitaxel, gemcitabine, and radiotherapy.

In some embodiments, small molecule drugs include, but are not limited to, pattern recognition receptor agonists other than the Toll-like receptor agonists provided in the present disclosure, anthracyclines, camptothecins, immunogenic cell death (ICD) inducers, tyrosine kinase inhibitors, taxanes, Bruton's tyrosine kinase (BTK) inhibitors, PI3K inhibitors, HDAC inhibitors, ERK inhibitors, MAPK inhibitors, PD-1/PD-L1 inhibitors, CTLA-4 inhibitors, TIGIT inhibitors, TIM3 inhibitors, EGFR inhibitors, VEGF inhibitors, PARP inhibitors, Her2, LAG-3, TNFR2 inhibitors, etc.

In some embodiments, anthracyclines include, but are not limited to: doxorubicin, epirubicin, pirarubicin, daunorubicin, aclarubicin, idarubicin, amrubicin, etc.

In some embodiments, camptothecins include, but are not limited to: topotecan, irinotecan, belotecan, exatecan (DX-8951), lurtotecan, sinotecan, rubitecan (9-NC), 9-aminocamptothecin (9-AC), gimatecan, karenitecin, DB-67, etc.

In some embodiments, tyrosine kinase inhibitors include, but are not limited to: sorafenib, sunitinib, gefitinib, erlotinib, lapatinib, afatinib, dacomitinib, vandetanib, neratinib, pelitinib (EKB-569), canertinib (CI-1033), osimertinib, rociletinib (CO-1686, Clovis Oncology), olmutinib (HM61713, Hanmi Pharmaceutical), naquotinib (ASP8273, Astellas), tesevatinib (XL647/KD019, Kadmon Corporation), nazartinib (EGF816, Novartis), and PF-06747775 (Pfizer), etc.

In some embodiments, immunogenic cell death inducers include, but are not limited to: DNA-damaging agents, bleomycin, etc.

In some embodiments, taxanes include, but are not limited to: paclitaxel, docetaxel, etc.

Pattern recognition receptor agonists other than the Toll-like receptor agonists provided in the present disclosure include, but are not limited to, agonists of Toll-like receptors, RIG-I-like receptors, Nod-like receptors, AIM2-like receptors, C-type lectin receptors, cGAS, and other intracellular DNA sensors.

In some embodiments, resiquimod (a TLR7/8 agonist, also known as R848) can be co-encapsulated with the CpG-ODNs provided in the present disclosure to form a dual-active-ingredient liposome (Tritolimod). This can further enhance immune responses in the tumor microenvironment, thereby effectively improving tumor treatment efficacy.

In some embodiments, the molar ratio of resiquimod to CpG-ODN is (0.1-5): 1, preferably 1:2.

In some embodiments, the chemotherapeutic drug doxorubicin hydrochloride (DOX) can be co-encapsulated with the CpG-ODNs provided in the present disclosure to form a dual-active-ingredient liposome (Doxotolimod). By promoting tumor antigen release via doxorubicin, the recognition and killing of tumor cells by CD8⁺ T cells activated by CpG-ODNs can be enhanced, thereby effectively improving tumor treatment efficacy.

In some embodiments, the molar ratio of doxorubicin to CpG-ODNs is (0.1-5): 1, preferably 1:2.

### Delivery System

The present disclosure provides a delivery system characterized by comprising an active ingredient and a lipid component, wherein the active ingredient comprises the Toll-like receptor agonist provided in the present disclosure.

In some embodiments, the mass ratio of the lipid component to the Toll-like receptor agonist provided in the present disclosure is (8-12):1, preferably 10:1.

In some embodiments, the lipid component comprises one or more of the following: cationic polymers, lipid nanoparticles (LNP), cationic liposomes, QTsome, lipopolyplexes, microparticles, microspheres, and nanoemulsions.

In some embodiments, the lipid component comprises one or more of cationic lipids, ionizable lipids, neutral lipids, and PEGylated lipids. In some embodiments, the PEGylated lipid can be at least one selected from the group consisting of DSG-PEG and DMG-PEG.

In some embodiments, the lipid component comprises DOTAP, DODMA, DSPC, cholesterol, and DMG-PEG. In some preferred embodiments, the lipid component comprises DOTAP, DODMA, DSPC, cholesterol, and DMG-PEG, and the molar ratio of DOTAP:DODMA:DSPC:cholesterol:DMG-PEG is (1-30):(5-50):(5-40):(15-60):(0-10). In some more preferred embodiments, the lipid component comprises DOTAP, DODMA, DSPC, cholesterol, and DMG-PEG, and the molar ratio of DOTAP: DODMA: D SPC: cholesterol: DMG-PEG is 15:25:20:38.5:1.5.

In some embodiments, the active ingredient in the delivery system comprises the Toll-like receptor agonist provided in the present disclosure and an additional therapeutic agent. In some preferred embodiments, the molar ratio of the additional therapeutic agent to the Toll-like receptor agonist provided in the present disclosure is (0.1-5): 1, preferably 1:2.

In some embodiments, the additional therapeutic agent is one or more agents selected from the group consisting of antibodies, chemotherapeutic drugs, and small molecule drugs. In some embodiments, the additional therapeutic agent is one or more agents selected from the group consisting of polyinosinic:polycytidylic acid (Poly(I:C)), STING agonists, imiquimod, resiquimod, gardiquimod, sorafenib, sunitinib, erlotinib, doxorubicin, paclitaxel, gemcitabine, and radiotherapy. In some preferred embodiments, the additional therapeutic agent is one or both of resiquimod and doxorubicin.

In some embodiments, resiquimod (a TLR7/8 agonist, also known as R848) can be co-encapsulated with the CpG-ODNs provided in the present disclosure to form a dual-active-ingredient liposome (Tritolimod). This can further enhance immune responses in the tumor microenvironment, thereby effectively improving tumor treatment efficacy.

In some embodiments, the molar ratio of resiquimod to CpG-ODN is (0.1-5): 1, preferably 1:2.

In some embodiments, the chemotherapeutic drug doxorubicin hydrochloride (DOX) can be co-encapsulated with the CpG-ODNs provided in the present disclosure to form a dual-active-ingredient liposome (Doxotolimod). By promoting tumor antigen release via doxorubicin, the recognition and killing of tumor cells by CD8⁺ T cells activated by CpG-ODNs can be enhanced, thereby effectively improving tumor treatment efficacy.

In some embodiments, the molar ratio of doxorubicin to CpG-ODNs is (0.1-5): 1, preferably 1:2.

The delivery system provided in the present disclosure, with its specific lipid composition, can provide a stable coating for CpG-ODNs, protecting them from degradation by endogenous enzyme systems, improving the bioavailability of CpG-ODNs, and extending the *in vivo* half-life of the oligonucleotide. Simultaneously, it can improve drug particle size and cellular adhesion, achieving the goal of increasing drug retention within the tumor. Utilizing the delivery system provided in the present disclosure, combined with intratumoral administration, can effectively improve drug retention within the tumor and efficiently deliver it to tumor-infiltrating immune cells, enhancing immune response in tumor tissue and improving tumor treatment efficacy.

The delivery system provided in the present disclosure can effectively increase the infiltration of immune cells such as dendritic cells, macrophages, CD3+ T cells, CD8⁺ T cells, and NK cells at the tumor site, while promoting the release of factors such as interleukins, interferons, chemokines, and granzymes, achieving the goal of tumor immunotherapy. Traditional adjuvants like alum, squalene, and QS21 are microemulsions with local retention and sustained-release properties. While they can induce antigen-specific immunity with good safety, they cannot induce strong Type I immune responses. Traditional CpG adjuvants can induce the correct CD8+ T cell response, but they rapidly diffuse systemically, causing adverse side effects that hinder treatment. Furthermore, in traditional CpG adjuvants, the adjuvant and antigen are separate, inducing non-specific autoimmunity. The delivery system provided in the present disclosure induces the correct Type I immune response without inducing systemic cytokines, combining the advantages of previous adjuvants to induce antigen-specific immunity.

The delivery system containing CpG-ODNs provided in the present disclosure can effectively inhibit tumor growth and can be formulated with one or more pharmaceutically acceptable excipients into a medicament for application in tumor treatment.

The delivery system containing CpG-ODNs provided in the present disclosure can effectively prevent tumor cells from metastasizing to other tissues and organs or prevent tumor recurrence.

### Preparation Methods

The present disclosure provides a method for preparing an active ingredient encapsulated by a lipid component, characterized by comprising the following steps (a)-(f):
(a) preparing the Toll-like receptor agonist, CpG-ODNs, provided in the first aspect of the present disclosure as an acidic solution;
(b) preparing each component of the lipid components as a lipid ethanol solution;
(c) mixing and reacting the solutions prepared in steps (a) and (b) to form an initial lipid nanoparticle solution;
(d) optionally repeating steps (a)-(c) to obtain more initial lipid nanoparticle solutions;
(e) removing ethanol from the initial lipid nanoparticle solution, adjusting the active ingredient concentration and pH to obtain a lipid nanoparticle suspension;
(f) optionally mixing an additional therapeutic agent with the lipid nanoparticle suspension to prepare a multi-active-ingredient liposome.

In some embodiments, when preparing a lipid-encapsulated single-active-ingredient using the above method, step (f) is an optional step.

In some embodiments, when preparing a lipid-encapsulated multi-active-ingredient using the above method, step (f) is performed. In some embodiments, the additional therapeutic agent used in step (f) is preferably resiquimod or doxorubicin.

The present disclosure also provides a method for preparing a delivery system, comprising the following steps: preparing a Toll-like receptor agonist buffer solution using a buffer and the Toll-like receptor agonist disclosed herein; preparing a lipid ethanol solution using anhydrous ethanol and the lipid component; mixing the lipid ethanol solution and the Toll-like receptor agonist buffer solution at a mass ratio of lipid component to CpG-ODN of (8-12):1; removing ethanol, diluting, and adjusting the pH to 6.8-7.2.

### Compositions, Pharmaceutical Compositions

The present disclosure provides a composition characterized by comprising the active ingredient encapsulated by a lipid component provided in the present disclosure and an additional therapeutic agent. In some embodiments, the additional therapeutic agent is one or more agents selected from the group consisting of antibodies, chemotherapeutic drugs, and small molecule drugs. In some embodiments, the additional therapeutic agent is one or more agents selected from the group consisting of polyinosinic:polycytidylic acid (poly IC), STING agonists, imiquimod, resiquimod, gardiquimod, sorafenib, sunitinib, erlotinib, doxorubicin, paclitaxel, gemcitabine, and radiotherapy. In some preferred embodiments, the additional therapeutic agent is one or both of resiquimod and doxorubicin.

The present disclosure provides a pharmaceutical composition characterized by comprising the Toll-like receptor agonist provided in the present disclosure, the active ingredient encapsulated by a lipid component, the composition, or the delivery system, and a pharmaceutically acceptable carrier or excipient.

As used herein, the term "pharmaceutical composition" refers to a mixture of the Toll-like receptor agonist, active ingredient encapsulated by a lipid component, composition, or delivery system provided in the present disclosure with other chemical components such as carriers, stabilizers, diluents, dispersants, suspending agents, thickeners, and/or excipients. The pharmaceutical composition facilitates the administration of the Toll-like receptor agonist, active ingredient encapsulated by a lipid component, composition, or delivery system to an organism. There are various ways known in the art for administering pharmaceutical compositions, including, but not limited to, intratumoral injection, intravenous injection, subcutaneous injection, intramuscular injection, intra-arterial injection, intraperitoneal injection, intra-central-nervous-system injection (e.g., intrathecal injection), intravesical instillation, interventional therapy, oral, transdermal, pulmonary, ocular, and topical administration.

As used herein, the pharmaceutical composition can be formulated into a dosage form suitable for administration to a subject via the desired route of administration. The dosage forms include, but are not limited to, tablets, capsules, caplets, pills, lozenges, powders, syrups, elixirs, suspensions, solutions, emulsions, transdermal patches, suppositories, inhalants, creams, ointments, lotions, pastes, sprays, lyophilized formulations, injections, and gels, etc.

The term "pharmaceutically acceptable carrier" includes pharmaceutically acceptable materials, compositions, or carriers, such as liquid or solid fillers, diluents, excipients, solvents, or encapsulating materials, which are involved in carrying or transporting the Toll-like receptor agonist, active ingredient encapsulated by a lipid component, composition, delivery system provided in the present disclosure within or to a subject, enabling it to perform its intended function. Each salt or carrier must be "acceptable" in the sense of being compatible with the other ingredients of the formulation and not harmful to the subject. Some examples of materials that can serve as pharmaceutically acceptable carriers include: sugars such as lactose, glucose, and sucrose; starches such as corn starch and potato starch; cellulose and its derivatives such as sodium carboxymethylcellulose, ethyl cellulose, and cellulose acetate; powdered tragacanth; malt; gelatin; talc; excipients such as cocoa butter and suppository waxes; oils such as peanut oil, cottonseed oil, safflower oil, sesame oil, olive oil, corn oil, and soybean oil; glycols such as propylene glycol; polyols such as glycerol, sorbitol, mannitol, and polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethanol; phosphate buffer solutions; diluents; granulating agents; lubricants; binders; disintegrants; wetting agents; emulsifiers; coloring agents; release agents; coating agents; sweeteners; flavoring agents; perfuming agents; preservatives; antioxidants; plasticizers; gelling agents; thickeners; hardeners; setting agents; suspending agents; surfactants; humectants; carriers; stabilizers; and other non-toxic compatible substances used in pharmaceutical formulations, or any combination thereof.

### Vaccines, Vaccine Adjuvants

The CpG-ODNs provided in the present disclosure can also be applied in the fields of vaccines and vaccine adjuvants to enhance immune responses and increase antibody titers. In some embodiments, CpG-ODNs can be used as a main component in the preparation of vaccine adjuvants, where the CpG-ODNs may exist in the vaccine formulation in lipid-conjugated form, directly encapsulated form, or directly exist as free form in the vaccine formulation.

The present disclosure provides a vaccine adjuvant or a vaccine characterized by comprising the Toll-like receptor agonist provided in the present disclosure, the active ingredient encapsulated by a lipid component, the composition, the delivery system, or the pharmaceutical composition.

In some embodiments, the aforementioned vaccine or vaccine adjuvant is for vaccines or vaccine adjuvants targeting KRAS, RSV, TERT, TP53, hepatitis virus, personalized neoantigens, etc.

### Uses and Methods

The present disclosure provides the use of the Toll-like receptor agonist, active ingredient encapsulated by a lipid component, composition, delivery system, or pharmaceutical composition of the present disclosure in any one of the following (a)-(j):
(a) use in the manufacture of a medicament for preventing tumorigenesis;
(b) use in the manufacture of a medicament for treating a tumor;
(c) use in the manufacture of a medicament for inhibiting tumor growth;
(d) use in the manufacture of a medicament for preventing tumor cell metastasis;
(e) use in the manufacture of a medicament for preventing tumor recurrence;
(f) use in the manufacture of a vaccine;
(g) use in the manufacture of a medicament for treating or preventing an inflammation-related disease or an infectious disease;
(h) use in the manufacture of a medicament for immune activation;
(i) use in the manufacture of a medicament for upregulating cytokine expression;
(j) use for upregulating cytokine expression for non-therapeutic purpose.

In some embodiments, immune activation includes, but is not limited to, B cell pathway activation, T cell pathway activation, myeloid lymphocyte pathway activation, and lymphoid lymphocyte pathway activation. In some embodiments, immune activation includes, but is not limited to, activation of immune cells such as macrophages, plasmacytoid dendritic cells (pDCs), B cells, T cells, CD3+ T cells, CD8+ T cells, NK cells, and granulocytes.

In some embodiments, the medicament can be administered in various ways, including, but not limited to, intratumoral injection, intravenous injection, subcutaneous injection, intramuscular injection, intra-arterial injection, intraperitoneal injection, intra-central-nervous-system injection (e.g., intrathecal injection), intravesical instillation, interventional therapy, oral, transdermal, pulmonary, ocular, and topical administration.

In some embodiments, the tumor is selected from solid tumors or tumors into which drugs can be injected, such as at least one of head and neck cancer, esophageal cancer, oral cancer, nasopharyngeal cancer, thyroid cancer, lung cancer, gastric cancer, liver cancer, pancreatic cancer, spleen cancer, non-small cell lung cancer, colorectal cancer, colon cancer, intestinal cancer, kidney cancer, brain tumor, glioma, bladder cancer, prostate cancer, breast cancer, ovarian cancer, fallopian tube cancer, cervical cancer, uterine cancer, bone cancer, osteosarcoma, chondroma, liposarcoma, neuroblastoma, synovial sarcoma, astrocytoma, glioblastoma multiforme, anaplastic astrocytoma, peritoneal cavity cancer, soft tissue sarcoma, sarcoma, rhabdomyosarcoma, advanced myxoid disease, melanoma, and skin cancer, such as non-melanoma skin cancer.

Inflammation-related diseases include, but are not limited to, delayed-type hypersensitivity, allergic diseases, lupus, graft-versus-host disease, vasculitis caused by hepatitis C, type I diabetes, type II diabetes, multiple sclerosis, rheumatoid arthritis, alopecia areata, atherosclerosis, psoriasis, organ transplant rejection, Sjogren's syndrome, Behcet's disease, spontaneous abortion, atopic diseases, asthma, and inflammatory bowel disease.

Infectious diseases include, but are not limited to, fungal infections, bacterial infections, mycoplasma or chlamydia infections, viral infections, such as viral hepatitis.

The present disclosure further provides a method of any one of the following (a)-(h):
(a) a method for preventing tumorigenesis;
(b) a method for treating a tumor;
(c) a method for inhibiting tumor growth;
(d) a method for preventing tumor cell metastasis;
(e) a method for preventing tumor recurrence;
(f) a method for treating or preventing an inflammation-related disease or an infectious disease;
(g) a method for immune activation;
(h) a method for upregulating cytokine expression;
characterized by comprising administering a therapeutically effective amount of the Toll-like receptor agonist, active ingredient encapsulated by a lipid component, composition, delivery system, or pharmaceutical composition of the present disclosure to a subject in need thereof.

In some preferred embodiments, when preventing tumorigenesis, treating a tumor, inhibiting tumor growth, preventing tumor cell metastasis, or preventing tumor recurrence, the Toll-like receptor agonist or active ingredient encapsulated by a lipid component of the present disclosure is administered to a subject in need at a dose of at least 0.1 mg/kg (mpk) body weight, for example, 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg, 1 mg/kg, 1.1 mg/kg, 1.2 mg/kg, 1.3 mg/kg, 1.4 mg/kg, 1.5 mg/kg, 2 mg/kg, 3 mg/kg, 4 mg/kg, 5 mg/kg, 6 mg/kg, 7 mg/kg, 8 mg/kg body weight. In some preferred embodiments, when preventing tumorigenesis, treating a tumor, inhibiting tumor growth, preventing tumor cell metastasis, or preventing tumor recurrence, the Toll-like receptor agonist or active ingredient encapsulated by a lipid component of the present disclosure is administered to a subject in need at a frequency of at least once a week, for a total of at least 3 administrations, for example, once every 3 days for a total of 4 administrations, once every 3 days for a total of 5 administrations, once a week for a total of 3 administrations, once a week for a total of 4 administrations, once a week for a total of 5 administrations. In some preferred embodiments, the subject in need is a mouse. Based on the knowledge of the above dosing regimens and frequencies and in combination with routine technical means in the art, those skilled in the art can convert dosing regimens and frequencies for one animal, such as mice, to those for another animal, such as humans.

In some embodiments, the tumor is selected from solid tumors or tumors into which drugs can be injected, such as at least one of head and neck cancer, esophageal cancer, oral cancer, nasopharyngeal cancer, thyroid cancer, lung cancer, gastric cancer, liver cancer, pancreatic cancer, spleen cancer, non-small cell lung cancer, colorectal cancer, colon cancer, intestinal cancer, kidney cancer, brain tumor, glioma, bladder cancer, prostate cancer, breast cancer, ovarian cancer, fallopian tube cancer, cervical cancer, uterine cancer, bone cancer, osteosarcoma, chondroma, liposarcoma, neuroblastoma, synovial sarcoma, astrocytoma, glioblastoma multiforme, anaplastic astrocytoma, peritoneal cavity cancer, soft tissue sarcoma, sarcoma, rhabdomyosarcoma, advanced myxoid disease, melanoma, and skin cancer, such as non-melanoma skin cancer.

Inflammation-related diseases include, but are not limited to, delayed-type hypersensitivity, allergic diseases, lupus, graft-versus-host disease, vasculitis caused by hepatitis C, type I diabetes, type II diabetes, multiple sclerosis, rheumatoid arthritis, alopecia areata, atherosclerosis, psoriasis, organ transplant rejection, Sjogren's syndrome, Behcet's disease, spontaneous abortion, atopic diseases, asthma, and inflammatory bowel disease.

Infectious diseases include, but are not limited to, fungal infections, bacterial infections, mycoplasma or chlamydia infections, viral infections, such as viral hepatitis.

As used herein, the term "subject" includes animals, such as vertebrates, preferably mammals, such as dogs, cats, pigs, cattle, sheep, horses, rabbits, rodents (e.g., mice, rats, or guinea pigs), or primates (e.g., monkeys, gorillas, chimpanzees, and humans).

As used herein, the term "treat" or "treating" refers to alleviating or improving a disease or disorder (i.e., slowing or stopping the development of the disease or at least one clinical symptom thereof); or alleviating or improving at least one physical parameter or biomarker associated with the disease or disorder.

As used herein, the term "therapeutically effective dose" refers to an amount that, compared to a corresponding subject not receiving that amount, results in a benefit from or treatment of the disease, but this amount is sufficiently low within the scope of reasonable medical judgment to avoid serious side effects. The therapeutically effective dose of the Toll-like receptor agonist, active ingredient encapsulated by a lipid component, composition, delivery system, or pharmaceutical composition described herein will vary depending on factors such as the chosen Toll-like receptor agonist, active ingredient encapsulated by a lipid component, composition, delivery system, or pharmaceutical composition; the route of administration; the severity of the disease being treated; the age, body size, weight, and physical condition of the patient being treated; the medical history of the patient being treated; the duration of treatment; the nature of concurrent treatments; the desired therapeutic effect; etc., but can be determined by a person skilled in the art in a conventional manner.

As used herein, the term "prevent" includes providing prophylaxis against the occurrence or recurrence of a disease for individuals who may be predisposed to the disease but have not yet been diagnosed with the disease.

The present disclosure further provides the Toll-like receptor agonist, active ingredient encapsulated by a lipid component, composition, delivery system, or pharmaceutical composition of the present disclosure for use in any one of the following (a)-(h):
(a) preventing tumorigenesis;
(b) treating a tumor;
(c) inhibiting tumor growth;
(d) preventing tumor cell metastasis;
(e) preventing tumor recurrence;
(f) treating or preventing an inflammation-related disease or an infectious disease;
(g) immune activation;
(h) upregulating cytokine expression.

The various embodiments described above for the Toll-like receptor agonist, active ingredient encapsulated by a lipid component, and delivery system of the present disclosure are also applicable to the pharmaceutical compositions, compositions, uses, and methods of the present disclosure (as long as they are not inherently contradictory to each other), and thus the various embodiments formed by combination are considered part of the present disclosure.

### Examples

The following describes exemplary embodiments of the present application in conjunction with the figures, including various details of the embodiments of the present application to aid understanding. It should be understood that they are considered merely exemplary and are not intended to limit the scope of protection of the present application. The scope of protection of the present application is defined only by the claims. Therefore, those of ordinary skill in the art will recognize that various changes and modifications can be made to the embodiments described herein without departing from the scope of the present application. Similarly, descriptions of known functions and structures are omitted from the following description for clarity and conciseness.

The experimental methods in the following examples, unless otherwise specified, are conventional methods performed according to the techniques or conditions described in the literature in the field or according to product instructions. The materials, reagents, etc., used in the following examples, unless otherwise specified, are commercially available.

### Example 1. Effect of CpG-ODNs on Tumor Growth in Mouse Colon Cancer

The test drugs were the four CpG-ODNs (T0009-1 to T0009-4) described in Table 1, and all phosphodiester bonds in the sequences shown in Table 1 were phosphorothioate-modified.

**Table 1. CpG-ODN Sequence Information**

| CpG-ODN Name | SEQ ID NO | Sequence (5'-3') |
|---|---|---|
| T0009-1 | 1 | TCGAACGTTCGAACGTTCGAACGTAT |
| T0009-2 | 2 | TCGAACGTTCGAACGTTCGAAT |
| T0009-3 | 3 | TCGAACGTTCGAACGTTCGAACGTTCGAACGTAT |
| T0009-4 | 4 | TCGAACGTTCGAACGTTCGAACGTTCGAACGTTCGAAT |

The above test drugs were diluted to 1 µg/µL using physiological saline (0.9% sodium chloride aqueous solution).

### 1. Experimental Protocol

(1) Fifty 7-week-old C57BL/6 mice (Vital River, equal numbers of males and females) were acclimated for one week, then weighed.
(2) The mouse colon cancer MC38 cell line (Nanjing Cobioer, Cat. No. CBP60825) was revived and passaged. When the cells were in good condition, MC38 cells (inoculum: 10⁶ cells) were transplanted subcutaneously into the backs of the mice. Changes in mouse body weight and tumor size were recorded.
(3) When the tumors reached approximately 100 mm³, the mice were grouped. Mice with excessively large or small tumors were excluded, and the remaining mice, equal to the number required for grouping, were randomly divided into 5 groups using a complete randomization method. The groups were designated: T0009-1 group, T0009-2 group, T0009-3 group, T0009-4 group, and saline group, with 7 mice per group.
(4) (Day 0) Each mouse received an intratumoral injection of 25 µL of T0009-1, T0009-2, T0009-3, T0009-4 or saline (0.9% sodium chloride aqueous solution). The dose was 1 mg of drug per kg body weight (1 mg/kg). Injections were given once every 3 days for a total of 5 times. The day of the first injection was designated Day 0.
(5) The growth and proliferation of the subcutaneous colon tumors in the drug administration groups and the saline control group were observed and recorded daily, including measuring mouse body weight and tumor volume. Tumor volume change curve and body weight change curve were plotted.
(6) On Day 18, the mice were euthanized. The tumors were excised, tumor volume was measured, and the net tumor weight was recorded. The tumor growth inhibition rate (TGI) was calculated based on the relative tumor volume (RTV) of each group on Day 18 relative to the grouping time. The formula for calculating TGI (%) is: TGI (%) = [1 - RTV (treatment group) / RTV (control group)] * 100%.

### 2. Experimental Results

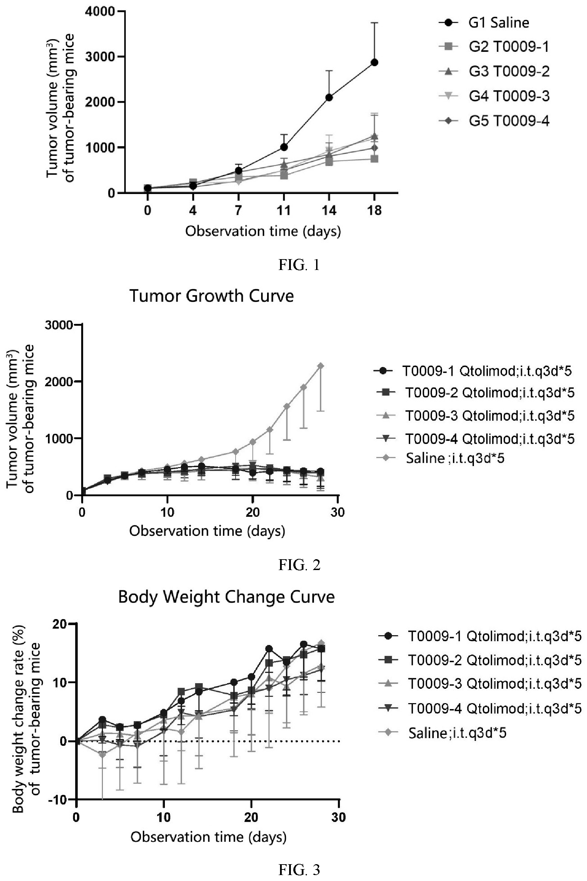

As shown in Fig. 1, all treatment groups showed very significant tumor suppression effects, with T0009-1 showing relatively better efficacy. The calculated TGI% values for T0009-1 to T0009-4 were 73.72%, 56.12%, 58.41%, and 65.37%, respectively, with T0009-1 having the best tumor inhibition efficacy. No deaths occurred in any treatment group from the start of administration until euthanasia on Day 18.

### Examples 2-5. Preparation of CpG-ODN-Encapsulating Lipid Nanoparticles (Qtolimods)

The CpG-ODNs used in Examples 2-5 were T0009-1, T0009-2, T0009-3, and T0009-4, respectively. The CpG-ODN Qtolimods prepared in Examples 2-5 were T0009-1 Qtolimod, T0009-2 Qtolimod, T0009-3 Qtolimod, and T0009-4 Qtolimod, respectively.

The method for preparing CpG-ODN-encapsulating Qtolimod included the following steps:
(1) CpG-ODN was taken and prepared into a CpG-ODN sodium acetate solution (pH=5.0) using sodium acetate buffer (pH=5.0), with a CpG-ODN concentration of 0.25 mg/mL and a sodium acetate concentration of 25 mM.
(2) DOTAP, DODMA, DSPC, cholesterol, and DMG-PEG were taken and prepared into a lipid ethanol solution using anhydrous ethanol, with a total lipid concentration of 7.5 mg/mL. The molar ratio of DOTAP:DODMA:DSPC:cholesterol:DMG-PEG was 15:25:20:38.5:1.5.
(3) 0.75 mL of the CpG-ODN sodium acetate solution from step (1) and 0.25 mL of the lipid ethanol solution from step (2) were pre-warmed at 25°C in a water bath. Then, the lipid ethanol solution was injected into the CpG-ODN sodium acetate solution. The mixture was incubated for 1 minute under magnetic stirring, then diluted 4-fold with pH=5.0, 25 mM sodium acetate buffer to obtain the initial Qtolimod solution (QTsome-encapsulated CpG-ODN). Steps (1)-(3) could be repeated and the products combined to obtain more CpG-ODN-loaded lipid nanoparticle (LNP) initial solution.
(4) Ethanol was removed from the LNP initial solution by ultrafiltration using a 100 kD molecular weight cut-off ultrafiltration tube at 2000 g. The nucleic acid concentration was adjusted to an appropriate level using 25 mM Tris buffer, and the pH was adjusted to 7.0 using 0.1 M sodium hydroxide solution to obtain suspensions of CpG-ODN-loaded LNPs, i.e., CpG-ODN Qtolimod suspensions. For *in vivo* and *in vitro* control experiments, the QTsome group used a lipid solution. The lipid solution in the QTsome group, and the lipid solution used in Example 14, was prepared by directly subjecting the lipid ethanol solution from step (2) to the ultrafiltration (ethanol removal) and pH adjustment steps of this step, without undergoing the mixing step with CpG-ODN described in step (3).

### Example 6. Preparation of Dual-Active-Ingredient Liposome Co-Encapsulating

### Resiquimod and CpG-ODN (Tritolimod)

According to a molar ratio of CpG-ODN to Resiquimod (Selleck, Cat. No. S8133) of 2:1, the T0009-1-loaded LNP suspension (T0009-1 Qtolimod suspension) prepared in Example 2 was mixed with Resiquimod to form the dual-active-ingredient liposome Tritolimod. Resiquimod has a high logP value and can actively distribute into the interior of the LNPs.

### Example 7. Preparation of Dual-Active-Ingredient Liposome Co-Encapsulating

### Doxorubicin (DOX) and CpG-ODN (Doxotolimod)

According to a molar ratio of CpG-ODN to DOX (Selleck, Cat. No. S1208) of 2:1, the T0009-1-loaded LNP suspension (T0009-1 Qtolimod suspension) prepared in Example 2 was mixed with DOX to form the dual-active-ingredient liposome Doxotolimod. DOX can be remotely loaded into the interior of the LNPs through binding with CpG and/or the trapping agent (e.g., acetate).

### Example 8. Effect of Qtolimods Prepared from Different CpG-ODNs on Tumor Growth in Mouse Colon Cancer

The test drugs were the Qtolimods prepared in Examples 2-5. The formulations were aliquoted in advance and stored at -20°C, then reconstituted on the day of use.

### 1. Experimental Protocol

(1) Fifty 7-week-old C57BL/6 mice (Vital River, equal numbers of males and females) were acclimated for one week, then weighed.
(2) The mouse colon cancer MC38 cell line (Nanjing Cobioer, Cat. No. CBP60825) was revived and passaged. When the cells were in good condition, MC38 cells (inoculum: 10⁶ cells) were transplanted subcutaneously into the backs of the mice. Changes in mouse body weight and tumor size were recorded.
(3) When the tumors reached approximately 100 mm³, the mice were grouped. Mice with excessively large or small tumors were excluded, and the remaining mice, equal to the number required for grouping, were randomly divided into 5 groups using a complete randomization method. The groups were designated: T0009-1 Qtolimod group, T0009-2 Qtolimod group, T0009-3 Qtolimod group, T0009-4 Qtolimod group, and saline group, with 7 mice per group.
(4) (Day 0) Each mouse received an intratumoral injection of 100 µL (administered at one or two sites as needed) of T0009-1 Qtolimod, T0009-2 Qtolimod, T0009-3 Qtolimod, T0009-4 Qtolimod, or saline (0.9% sodium chloride aqueous solution). The dose was 1 mg/kg. Injections were given once every 3 days for a total of 5 times. The day of the first injection was designated Day 0.
(5) The growth and proliferation of the subcutaneous colon tumors in the drug administration groups and the saline control group were observed and recorded daily, including measuring mouse body weight and tumor volume. Tumor volume change curve and body weight change curve were plotted.
(6) On Day 30, the mice were euthanized. The tumors were excised, tumor volume was measured, and the net tumor weight was recorded. The tumor growth inhibition rate was calculated.

### 2. Experimental Results

As shown in Figs. 2-3, obvious efficacy began to appear in all treatment groups around experimental Day 8. By Day 30, the tumor volume in the saline control group reached the humane endpoint (2500 mm³), while all Qtolimod treatment groups showed very significant tumor suppression effects. The calculated TGI% values for T0009-1 to T0009-4 Qtolimods were 84.42%, 85.52%, 89.15%, and 85.80%, respectively. No deaths occurred in any treatment group from the start of administration until euthanasia on Day 30.

Throughout the experimental period, there were no significant abnormalities in body weight or clinical status in either the saline control group or the treatment groups, indicating that the animals tolerated the test drug doses well under these tumor model conditions.

In summary, T0009-1 Qtolimod, T0009-2 Qtolimod, T0009-3 Qtolimod, and T0009-4 Qtolimod exhibited significant inhibitory effects on the growth of mouse transplanted tumors, and the animals showed good tolerance. There were no significant differences among the treatment groups in terms of improving the survival of tumor-bearing animals, tumor suppression, and clinical symptoms.

### Example 9. Effect of Qtolimods Prepared from Different CpG-ODNs on Tumor Growth in Mouse Skin Melanoma

The test drugs were the Qtolimods prepared in Examples 2-5. The formulations were aliquoted in advance and stored at -20°C, then reconstituted on the day of use.

### 1. Experimental Protocol

(1) Fifty 7-week-old C57BL/6 mice were acclimated for one week, then weighed.
(2) The inoculation conditions are shown in Table 2 below.

**Table 2. Cell Inoculation Information**

| Animal Strain | Number | Inoculated Cells | Inoculation Site | Inoculum | Matrigel Added? | Inoculation Volume |
|---|---|---|---|---|---|---|
| C57BL/6 | 50 mice | B16F10 (Cell Bank of Chinese Academy of Sciences, Cat. No. TCM36) | Right shoulder, subcutaneous | 2×10⁵ cells | No | 0.1 mL |

(3) After inoculation, tumor volume and body weight were measured once a week. When the average tumor volume reached approximately 40-60 mm³, the mice were grouped. Mice with excessively large or small tumors were excluded, and the remaining mice, equal to the number required for grouping, were randomly divided into 5 groups using a complete randomization method. The groups were designated: T0009-1 Qtolimod group, T0009-2 Qtolimod group, T0009-3 Qtolimod group, T0009-4 Qtolimod group, and saline group, with 8 mice per group. Administration of the test drug or saline (0.9% sodium chloride aqueous solution) began immediately after grouping. Each mouse received an intratumoral injection (i.t.) of 50 µL (administered at one or two sites as needed) at a dose of 1 mg/kg. Injections were given once every 3 days for a total of 5 times. The day of the first injection was designated Day 0.
(4) After administration began, body weight and tumor volume were measured three times per week. When the tumor volume of any single animal reached 3000 mm³, that animal was humanely euthanized, considered a death. The median survival time (days, MST) for each group was calculated, survival curves were plotted, and statistical comparisons were made.

### 2. Experimental Results

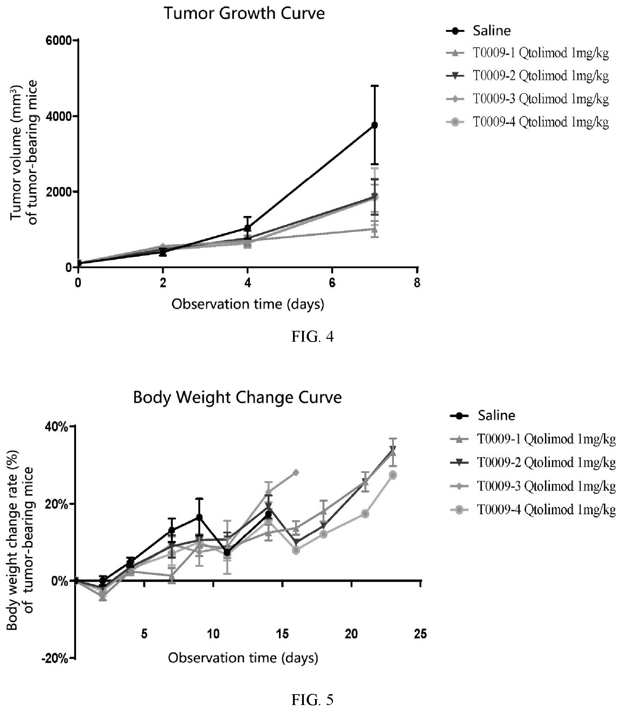

As shown in Figs. 4-6, obvious efficacy appeared in all treatment groups by experimental Day 7, with the T0009-1 Qtolimod group showing relatively better tumor suppression.

Analyzing the average survival time of each group, the average survival of the saline control group was 9 days, while for the T0009-1 to T0009-4 Qtolimod groups, it was 16, 12, 11, and 12 days, respectively. The calculated TGI% for the T0009-1 Qtolimod group was 75%, showing a highly significant difference compared to the saline control group (P-values were all less than 0.01). Moreover, the animals in the T0009-1 Qtolimod group had the longest survival time among all treatment groups.

Throughout the experimental period, there were no significant abnormalities in body weight or clinical status in either the saline control group or the treatment groups, indicating that the animals tolerated the test drug doses well under these tumor model conditions.

In summary, T0009-1 Qtolimod, T0009-2 Qtolimod, T0009-3 Qtolimod, and T0009-4 Qtolimod exhibited significant inhibitory effects on the growth of mouse transplanted tumors, and the animals showed good tolerance. Among them, T0009-1 Qtolimod performed best in improving the survival of tumor-bearing animals, tumor suppression, and clinical symptoms.

### Example 10. Effect of Dual-Active-Ingredient Liposomes on Tumor Growth in Mouse Colon Cancer

The test drugs were T0009-1 Qtolimod prepared in Example 2, the dual-active-ingredient liposome Tritolimod prepared in Example 6, and the dual-active-ingredient liposome Doxotolimod prepared in Example 7. The formulations were aliquoted in advance and stored at -20°C, then reconstituted on the day of use.

### 1. Experimental Protocol

Same as the experimental protocol in Example 8.

### 2. Experimental Results

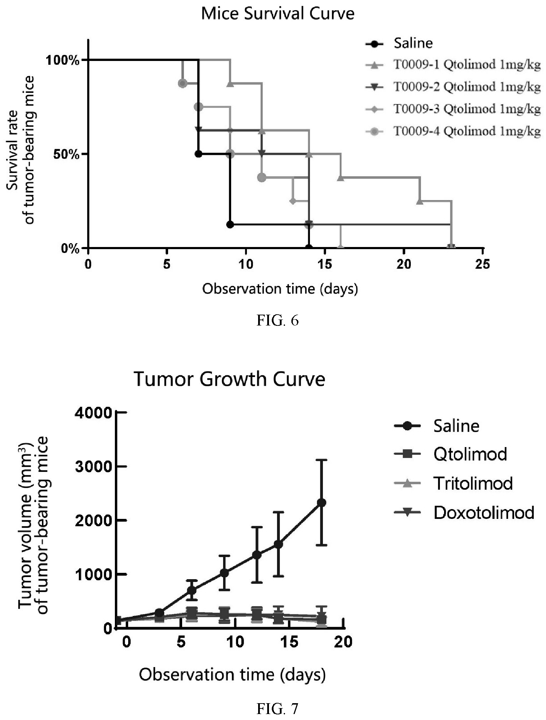

As shown in Figs. 7-8, obvious efficacy began to appear in all treatment groups by experimental Day 6. By Day 12, the Qtolimod group (receiving T0009-1 Qtolimod), the Tritolimod group (receiving Tritolimod), and the Doxotolimod group (receiving Doxotolimod) all showed very significant (P<0.001) tumor suppression effects. The calculated TGI% values for the Qtolimod, Tritolimod, and Doxotolimod groups were 90.97%, 94.09%, and 91.03%, respectively. Throughout the experimental period, there were no significant abnormalities in body weight or clinical status in either the saline control group or the treatment groups, indicating that the animals tolerated the test drug doses well under these tumor model conditions.

### Example 11. Effect of Systemic Administration of the Dual-Active-Ingredient Liposome Tritolimod on Tumor Growth in Mouse Skin Melanoma

The test drug was the dual-active-ingredient liposome Tritolimod prepared in Example 6. The formulation was aliquoted in advance and stored at -20°C, then reconstituted on the day of use.

### 1. Experimental Protocol

Similar to Example 9, except that this example used tail vein injection as the route of administration.

### 2. Experimental Results

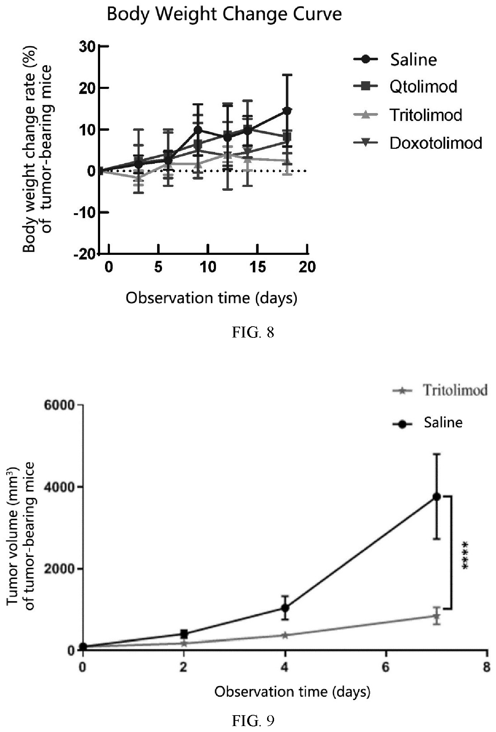

As shown in Figs. 9-10, systemic administration of Tritolimod could effectively inhibit tumor growth in tumor-bearing mice. The calculated TGI for the treatment group reached 80%, showing a significant difference compared to the saline control group (P<0.001). Moreover, the survival period of the mice in the treatment group was effectively extended.

### Example 12. Study on Dose-Dependent Anti-Tumor Efficacy and Prevention of Recurrence

This example used intratumoral administration to study the effect of T0009-1 Qtolimod on tumor growth. The test drug was the lipid nanoparticle T0009-1 Qtolimod prepared in Example 2. The formulation was aliquoted in advance and stored at -20°C, then reconstituted on the day of use.

### 1. Experimental Protocol

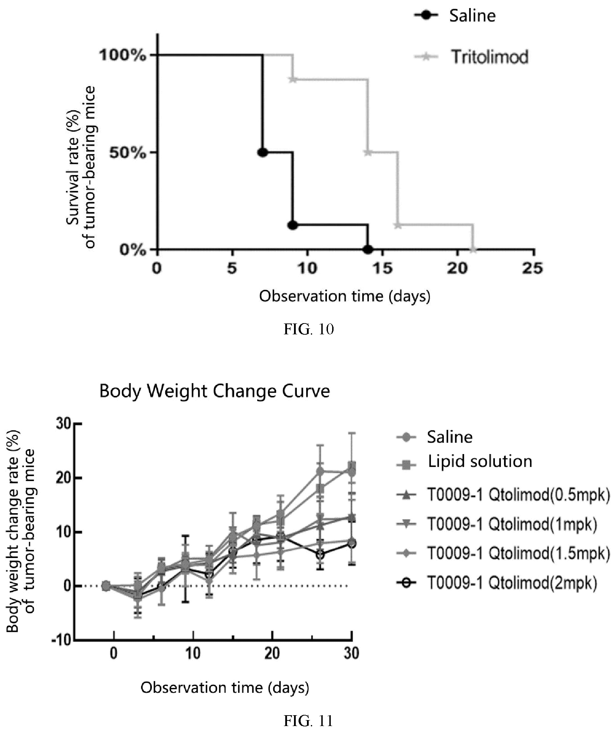

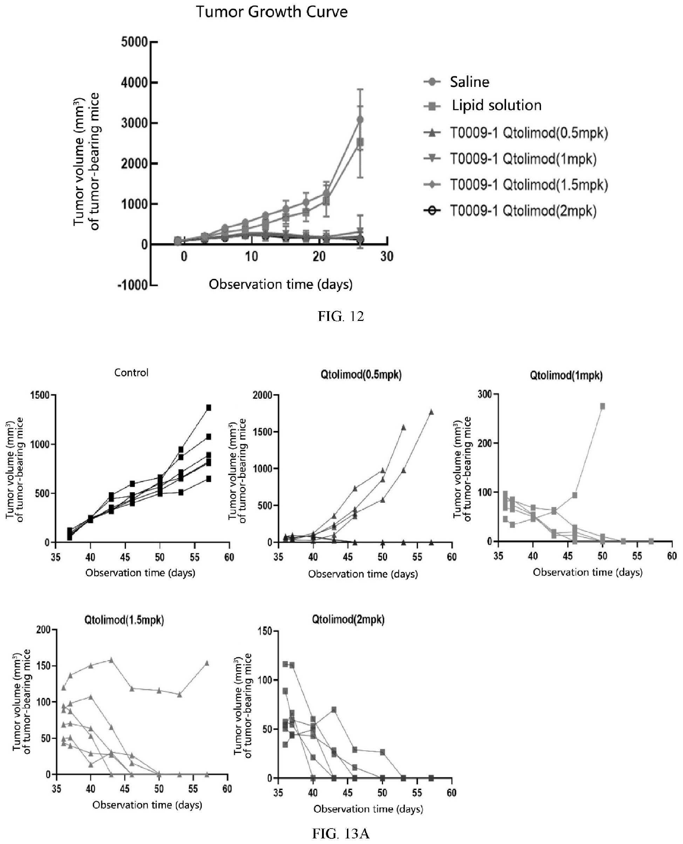

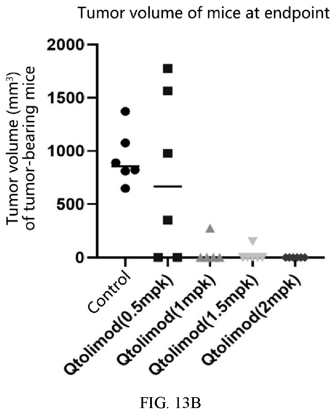

(1) MC38 cells were revived and cultured *in vitro* to obtain 5×10⁶ cells.
(2) Thirty-six 7-week-old female C57BL/6 mice were acclimated for one week, then weighed.
(3) The mouse colon cancer MC38 cell line was revived and passaged. When the cells were in good condition, MC38 cells (inoculum: 10⁶ cells) were transplanted subcutaneously into the right shoulder of the mice. Changes in mouse body weight and tumor size were recorded.
(4) When the tumors reached approximately 100 mm³, the mice were grouped. Mice with excessively large or small tumors were excluded, and the remaining mice, equal to the number required for grouping, were randomly divided into 6 groups using a complete randomization method. The groups were designated: QTsome group, T0009-1 Qtolimod (0.5 mg/kg) group, T0009-1 Qtolimod (1 mg/kg) group, T0009-1 Qtolimod (1.5 mg/kg) group, T0009-1 Qtolimod (2 mg/kg) group, and saline control group, with 6 female mice per group. The QTsome group received the lipid solution described in step (4) of Example 2, and the control group received 0.9% sodium chloride aqueous solution.
(5) (Day 0) Each mouse received an intratumoral injection of 50 µL (administered at one or two sites as needed) of the respective T0009-1 Qtolimod dose (0.5, 1, 1.5, or 2 mg/kg), the lipid solution, or saline (0.9% sodium chloride aqueous solution). Injections were given once every 3 days for a total of 5 times. The day of the first injection was designated Day 0.
(6) The growth and proliferation of the right shoulder subcutaneous colon tumors in the drug administration groups and the control group were observed and recorded daily, including measuring mouse body weight and tumor volume. The tumor growth inhibition rate was calculated, and tumor volume change curves were plotted (results shown in Figs. 11-12).
(7) To understand whether the drug inhibits tumor recurrence, on Day 36, MC38 cells (inoculum: 10⁶ cells) were subcutaneously implanted into the left shoulder of mice in the T0009-1 Qtolimod (0.5 mg/kg, i.e., 0.5 mpk) group, T0009-1 Qtolimod (1 mpk) group, T0009-1 Qtolimod (1.5 mpk) group, and T0009-1 Qtolimod (2 mpk) group. Since animals in the saline control and QTsome groups had died, a new control group was established for this study. The new control group consisted of 6 female C57BL/6 mice of the same age as the mice in the T0009-1 Qtolimod groups. The same number of MC38 cells were implanted at the same site (left shoulder) in these 6 female mice, but they were not administered any drug. Tumor growth was monitored in all mice after tumor inoculation, and tumor growth curves were plotted (results shown in Figs. 13A-B).

### 2. Experimental Results

In the *in vivo* efficacy experiment using the syngeneic model of female C57BL/6 mice implanted with mouse colorectal cancer MC38 cells, obvious efficacy began to appear in all Qtolimod groups by experimental Day 6. The calculated TGI% for all Qtolimod groups was greater than 63%. By Day 26, tumor volume in the saline control group reached the humane endpoint, while all Qtolimod treatment groups (0.5, 1, 1.5, 2 mpk) showed very significant tumor suppression effects with no significant differences. The calculated TGI% values for the 0.5, 1, 1.5, and 2 mpk Qtolimod treatment groups were 96.20%, 92.45%, 97.73%, and 98.82%, respectively. By monitoring tumor changes in each mouse, by Day 56, 1/2 of the mice in the 0.5 mpk group died due to re-worsening of the right shoulder tumor, while 1/3 of the mice had continuously shrinking right tumors. In the 1.0 mpk group, 1/3 of the mice died due to re-worsening of the right shoulder tumor, while the remaining 2/3 had continuously shrinking right tumors. In the 1.5 mpk and 2.0 mpk groups, no mice died due to tumor worsening. In the 1.5 mpk group, the right shoulder tumors were completely eliminated in 5 mice, and in the 2 mpk group, the right shoulder tumors were completely eliminated in all 6 mice. The above data show a clear dose-dependent relationship between animal survival/tumor elimination rate and drug dose. For the simulated tumor recurrence study, except for some mice in the 0.5 and 1 mpk groups that died due to right tumor growth, tumors implanted on the left side in almost all treatment group mice grew slowly and then gradually disappeared. This suggests that mice treated for the right tumor developed tumor-specific memory immune cells (especially CD8⁺ T cells) capable of recognizing and clearing the same type of tumor cells at other locations.

### Example 13. Comparison of Intratumoral Content after Intratumoral Injection of Qtolimod vs. Free CpG ODN

The test drugs in this experiment were T0009-1 Qtolimod prepared according to the method in Example 2 and free T0009-1. This experiment used intratumoral administration to study drug retention within tumor tissue. The T0009-1 (CpG ODN) used in both T0009-1 Qtolimod and free T0009-1 was labeled with Cy5. The formulations were aliquoted in advance and stored at - 20°C, then reconstituted on the day of use.

### 1. Experimental Protocol

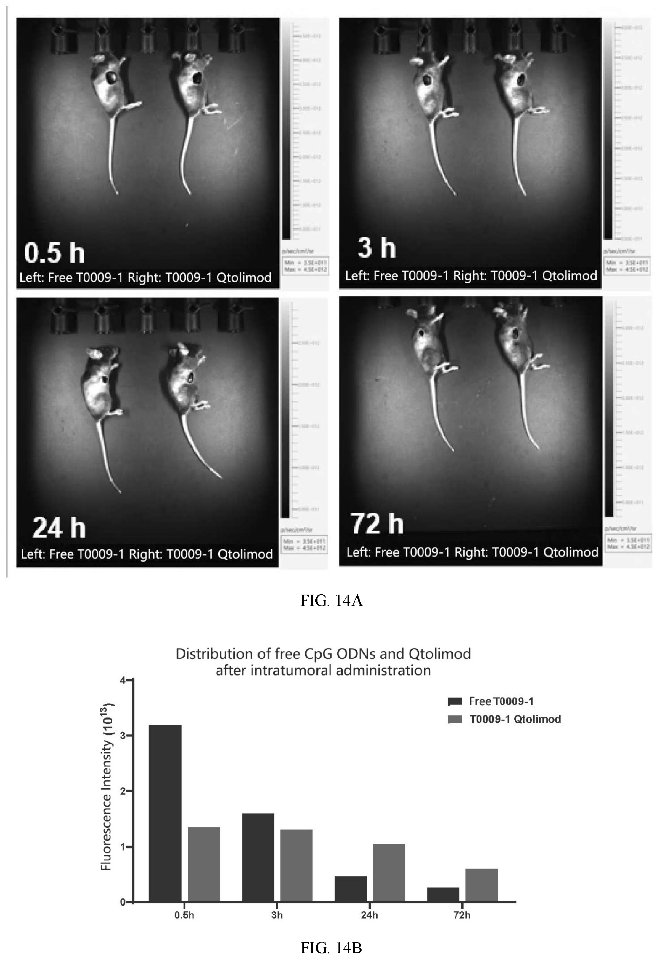

(1) MC38 cells were revived and cultured *in vitro* to obtain 5×10⁶ cells.
(2) Two 7-week-old female C57BL/6 mice were acclimated for one week, then weighed.
(3) The mouse colon cancer MC38 cell line was revived and passaged. When the cells were in good condition, MC38 cells (inoculum: 10⁶ cells) were transplanted subcutaneously into the right shoulder of the mice. Changes in mouse body weight and tumor size were recorded.
(4) When the tumors reached approximately 150 mm³, the mice were divided into the T0009-1 Qtolimod group and the free T0009-1 group and received an intratumoral injection (1 mg/kg).
(5) At 0.5 h, 3 h, 1 day, and 3 days after administration, *in vivo* imaging was performed using a Tanon (ABL-X) system (excitation 630 nm, emission 699 nm). The imaging results are shown in Fig. 14A.

### 2. Experimental Results

Using Cy5 labeling, the retention of free T0009-1 vs. T0009-1 Qtolimod within the tumor was compared. According to Figs. 14A-B, the content of free T0009-1 in tumor tissue decreased rapidly after intratumoral administration. It decreased to 50% by 3h and was only 8% remaining by 72h. In contrast, the content of T0009-1 Qtolimod in tumor tissue showed essentially no reduction at 3h, followed by a 20% reduction by 24h, with about 50% of the drug still retained in the tumor tissue at 72h. The results indicate that QTsome encapsulation of CpG ODN can significantly increase drug retention at the tumor site, markedly reduce the proportion entering systemic circulation. This not only reduces systemic toxicity and improves drug safety but also ensures drug exposure at the tumor site, achieving a sustained-release effect and significantly enhancing tumor treatment efficacy.

### Example 14. Study on Immune Activation Mechanism of the Test Drug

The test drug in this example was T0009-1 Qtolimod from Example 2. Control groups used saline or the lipid solution described in step (4) of Example 2. The animal experiment protocol was the same as in Example 8.

### I. Immunohistochemistry Experiment

After completing all administrations, mouse tumors were dissected, and immunohistochemistry was performed as follows:

### 1. Sample Processing

The received tumor-bearing samples were further trimmed. Starting from the injection site, the tissue was cut in half along its largest surface for experimentation. Mouse tissues were dehydrated using a Leica automatic tissue processor (Leica, HistoCore PEGASUS).

### 2. Immunohistochemical Staining of Tumor-Bearing Tissues for F4/80, CD8 alpha, and CD11b

(1) Dehydrated tissues were paraffin-embedded, and tissue paraffin sections (4 µm thick, 5 sections per tissue) were prepared for immunohistochemistry.
(2) F4/80 (CST, 70076T), CD8 alpha (abcam, ab209775), and CD11b (abcam, ab133357) were used as primary antibodies for immunohistochemical staining of mouse tumor-bearing tissue paraffin sections. Processed tissue section samples were incubated with primary antibody at room temperature for 1 h, then washed 3 times for 5 minutes each on a shaker with 0.1% Triton TBS. Goat anti-rabbit secondary antibody (Peroxidase AffiniPure Goat Anti-Rabbit IgG (H+L) (Jackson Immuno, 111-035-003), diluted 1:1000 in PBS) was applied and incubated at room temperature for 30 min, followed by washing 3 times for 5 minutes each on a shaker with 0.1% Triton TBS.
(3) Substrate color development: Approximately 200 µL of DAB chromogen (Fuzhou Maixin, DAB-1031, DAB kit (20x)) was added, and color development proceeded at room temperature for 5 min, then immediately immersed in tap water to stop the reaction.
(4) Nuclear counterstaining and dehydration/mounting: Cell nuclei were counterstained with hematoxylin for 1 min, followed by dehydration through graded ethanol and xylene, and manual mounting with neutral balsam.
(5) All sections were digitally scanned (Olympus VS200). The digital quantitative analysis software QuPath was used to analyze tissue necrosis rate in scanned images of immunohistochemically (IHC) stained tissues (semi-automatically marking necrotic tissue areas lacking nuclei). QuPath was also used for statistical analysis of positive signals (brown) for F4/80, CD8 alpha, and CD11b.

### 3. Data Analysis

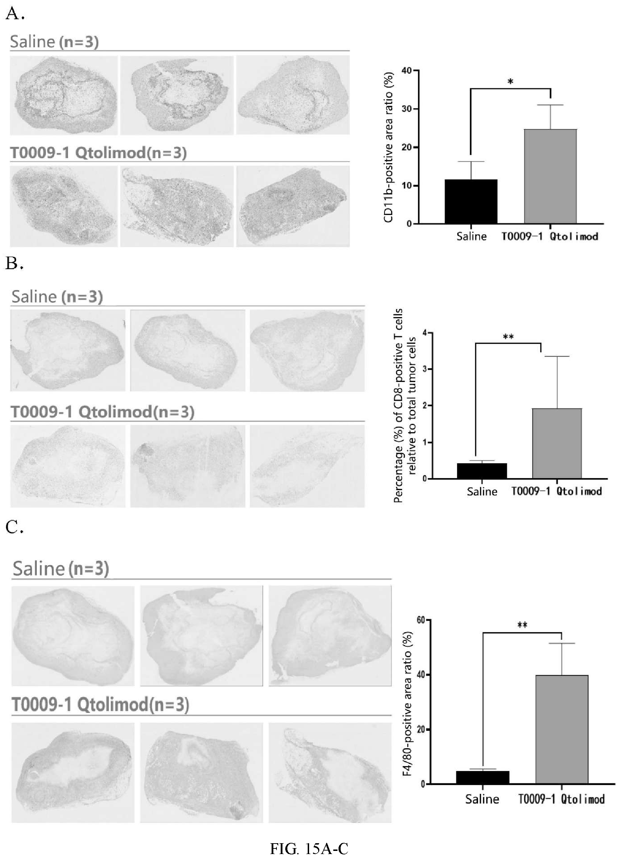

GraphPad Prism software was used to calculate Mean ± SEM. Difference significance testing used T-test. P < 0.05 was considered a significant difference between groups. Results are shown in Figs. 15A-C.

### II. ELISA Detection of Cytokines IL-12, IL-10, IFN-y, TNF-α, IL-6, IL-1β Expression in Mouse Blood and Tumor Tissue Samples after Administration

### 1. Sample Processing

After completion of administration with T0009-1 Qtolimod, saline, or lipid solution, blood was collected from the mouse heart. The blood was left at room temperature for 1h, then centrifuged at 5500 rpm for 20 min to collect serum samples. Tumors were dissected, minced with surgical scissors to approximately 1 mm³ pieces, then 2 mL PBS was added and vortexed. After centrifugation at 2000 rpm for 2 min, the supernatant was collected as the tumor sample.

### 2. ELISA Detection

ELISA kits were provided by QuanLab/CUSABIO. The experimental method was as follows:
(1) All reagents were brought to room temperature (18-25°C) and equilibrated for at least 30 min. Reagents were prepared according to the kit instructions for use. Sample addition: Blank wells (S0), standard wells (duplicate), and sample wells were set up. 100 µL of standard or test sample (serum sample, tumor sample) was added to each well. Blank wells (S0) received an equal volume of sample diluent. The plate was gently shaken to mix, covered with a plate sealer, and incubated at 37°C for 2 hours.
(2) Liquid was discarded, and the plate was dried by shaking without washing.
(3) 100 µL of biotin-labeled antibody working solution was added to each well. A new plate sealer was applied, and incubation proceeded at 37°C for 1 hour.
(4) Liquid in the wells was discarded, and the plate was dried by shaking. The plate was washed 3 times, soaking for 2 minutes each time with 200 µL/well, then dried by shaking.
(5) 100 µL of horseradish peroxidase-labeled avidin working solution was added to each well. A new plate sealer was applied, and incubation proceeded at 37°C for 1 hour.
(6) Liquid in the wells was discarded, and the plate was dried by shaking. The plate was washed 5 times, soaking for 2 minutes each time with 200 µL/well, then dried by shaking.
(7) 90 µL of substrate solution was added to each well in sequence, and the plate was incubated at 37°C protected from light for 15-30 minutes for color development.
(8) 50 µL of stop solution was added to each well in sequence to stop the reaction.
(9) Within 5 minutes after the reaction, the optical density (OD value) of each well was measured at 450 nm using a microplate reader.

### 3. Data Processing

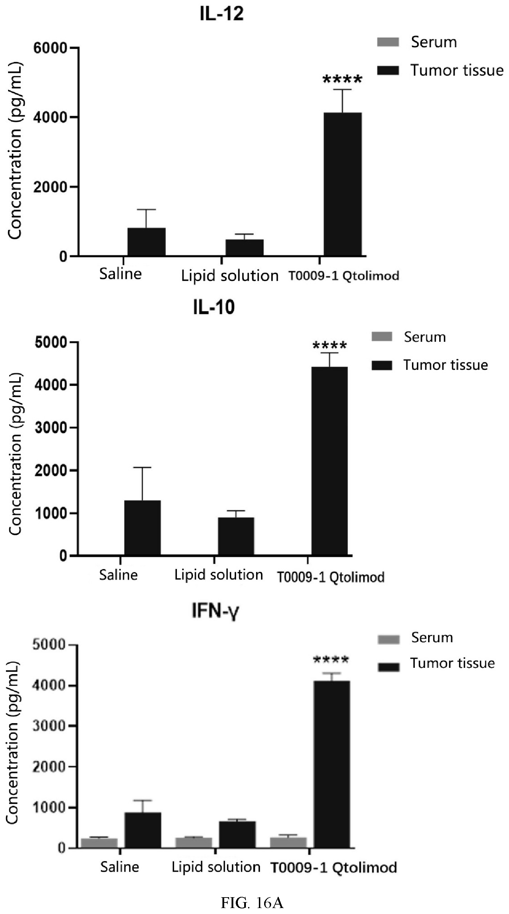

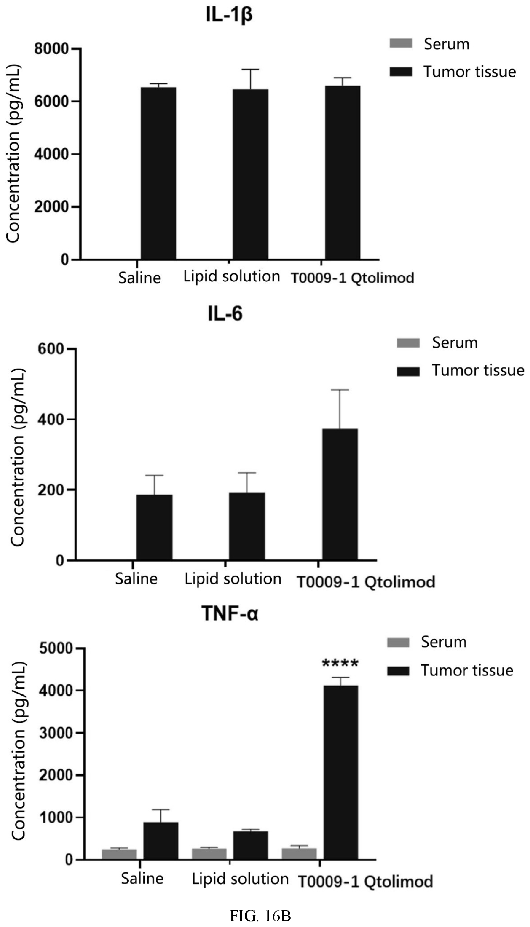

The S0 well value was subtracted from both standard and sample values to plot the curve, with duplicate wells averaged before calculation. After plotting the standard curve using relevant software, the measured OD values were substituted into the standard curve equation to calculate sample concentrations. Results are shown in Figs. 16A-B.

### III. Experimental Results

Immunohistochemistry results showed that compared to the saline control group, macrophage and CD8+ T cell infiltration at the tumor site significantly increased in the treatment group, playing roles in directly killing tumor cells, clearing tumor tissue debris, and stimulating adaptive immunity through antigen presentation. Infiltration of CD11b-positive cells (monocytes, granulocytes, activated lymphocytes, NK cells, dendritic cells) also increased, playing important roles in inducing efficient, specific cellular immune responses against tumor-associated antigens and immune cell recruitment. Cytokine detection results showed that compared to the saline and LNP control groups, IL-12, IL-10, IFN-γ, and TNF-α were significantly upregulated within tumor tissue in the Qtolimod treatment group, while IL-1β and IL-6 showed no significant change. All cytokine expression levels in the blood were below the detection limit. IL-12 promotes NK cell and CD8⁺ T cell proliferation and cytotoxic activity; induces Th1 differentiation and IFN-γ secretion; promotes antigen presentation. IL-10, produced by antigen-presenting cells, promotes CD8+ T cell activation and inhibits inflammatory cytokines like TNF-α, IL-6, and IL-1 via macrophage activation. IFN-γ is mainly produced by T cells, NK cells, and NKT cells, exerting its killing effects by inducing apoptosis or promoting non-apoptotic cell death, as well as indirectly by sensitizing tumor cells to apoptosis-inducing immune responses.

### Example 15. Study on the Impact of the Delivery System on Safety

To study the impact of the QTsome delivery system on the safety of the CpG ODN, this experiment set up a vehicle control group, QTsome group, active pharmaceutical ingredient (API) group, and Qtolimod group. The vehicle control group received saline (0.9% sodium chloride aqueous solution), the QTsome group received the lipid solution described in step (4) of Example 2, the API group received free T0009-1, and the Qtolimod group received T0009-1 Qtolimod. Mouse survival and body weight changes after administration were observed.

### 1. Experimental Protocol

(1) Each group consisted of 6 CD1 (ICR) mice aged 6-8 weeks (Vital River, equal numbers of males and females), weighing 20-30 g.
(2) On days 1, 8, 15, 22, and 29, saline, lipid solution, free T0009-1, or T0009-1 Qtolimod was administered via subcutaneous (SC) injection, once a week (QW), for a total of 5 administrations over 29 days. The dose was 20 mg/kg. The lipid content in the QTsome group was the same as that in the Qtolimod group. The day of the first subcutaneous injection was designated Day 1.

### 2. Experimental Results

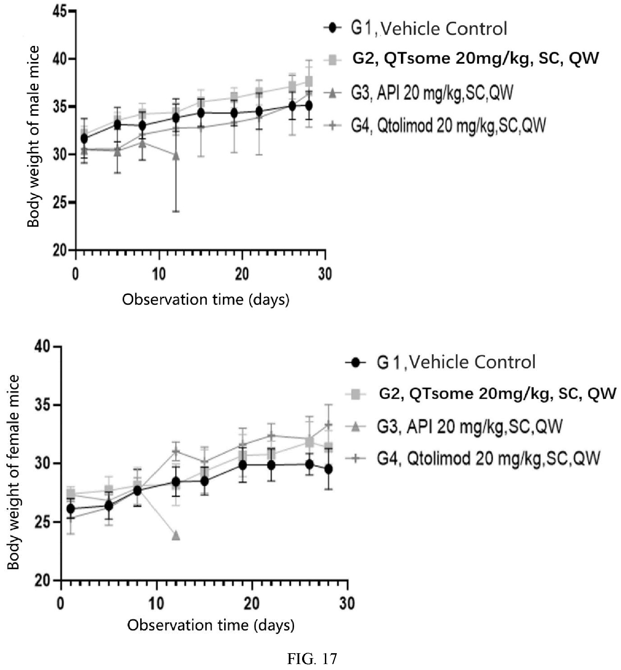

As shown in Fig. 17, mice in all groups except the API group tolerated the treatment well, with no significant body weight decrease observed. Mice receiving free T0009-1 showed body weight decrease after administration, and on day 12, 1 male and 2 female mice died, accounting for 50% of the group. Necropsy of the remaining non-dead mice in the API group revealed noticeably paler kidneys with slightly lower weight, and significantly enlarged liver and spleen. In males, liver weight was 2.5 times that of the vehicle control group, and spleen weight was 5.4 times that of the control. In females, liver weight was 3 times that of the control, and spleen weight was 6.8 times that of the control. Necropsy of Qtolimod group mice at the endpoint (after 5 administrations) revealed: in males, liver weight was 1.7 times that of the vehicle control, spleen weight was 2.9 times that of the control; in females, liver weight was 1.7 times that of the control, spleen weight was 3.4 times that of the control. Based on these experimental results, the QTsome delivery system can effectively improve the safety profile of this product.

### Example 16. Efficacy of the Test Drug in Treating Mouse Melanoma B16-Luc Metastasis Model

In this experiment, T0009-1 Qtolimod was prepared using the method described in Examples 2-5 to encapsulate T0009-1 with lipid nanoparticles. A mouse model of subcutaneous tumors and systemic metastasis was established by transplanting mouse melanoma B16-Luc cells subcutaneously and intravenously into each mouse to evaluate the therapeutic effect of the test drug on both subcutaneous and systemic metastasis models of B16-Luc syngeneic transplants.

### 1. Construction of Subcutaneous Tumor and Metastasis Models

Mouse melanoma B16-Luc cells were aliquoted into several 75 cm² flasks and cultured conventionally using DMEM medium containing 10% fetal bovine serum in a constant temperature and humidity incubator at 37°C, 5% CO2. When cells were in the logarithmic growth phase, they were digested with 0.25% trypsin, collected after detachment, centrifuged, supernatant removed, washed twice with PBS, and resuspended to the required cell concentration for animal transplantation. Cells were resuspended in sterile PBS buffer mixed 1:1 with Matrigel matrix to a concentration of 1×10⁷/mL, and 0.1 ml was implanted subcutaneously into the left axilla of C57BL/6J mice to construct the subcutaneous transplant tumor model. Simultaneously, cells were resuspended in sterile PBS buffer to 5×10⁶/mL, and 0.2 ml was injected intravenously via the tail vein into C57BL/6J mice to construct the systemic metastasis model.

### 2. Subcutaneous Tumor and Systemic Metastasis Experiment

T0009-1 Qtolimod formulation was aliquoted in advance, stored at -20°C, and reconstituted on the day of use. When subcutaneous tumor volume reached approximately 68-105 mm³, tumor-bearing mice with good tumor growth, no ulceration, and healthy status were selected. Mice with excessively large or small tumors were excluded, and the remaining mice, equal to the number required for grouping, were randomly divided into 2 groups using a complete randomization method. The groups were designated: T0009-1 Qtolimod 1.5 mg/kg group and saline control group, with 16 mice per group. The T0009-1 Qtolimod 1.5 mg/kg group received 1.5 mg/kg body weight of T0009-1 Qtolimod, and the control group received 0.9% sodium chloride aqueous solution. The grouping day was designated Day 1 (D1). Administration was via subcutaneous intratumoral injection at 2.5 ml drug/kg body weight, once every 3 days on D1, D4, D7, and D10, for a total of 4 times. The saline control group received an equal volume of saline.

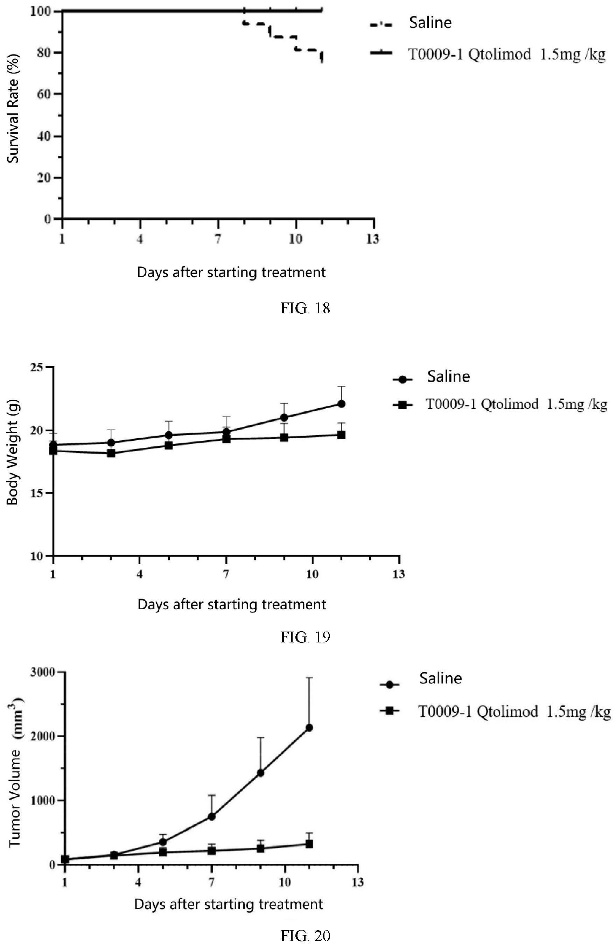

The experimental results showed that on D8, D9, D10, and D11 after administration, 1 animal died each day in the saline control group, while no deaths occurred in the T0009-1 Qtolimod 1.5 mg/kg group. Survival of animals in each group is shown in Fig. 18. Throughout the period from before administration, during administration, and until euthanasia, daily observations of all animals in the T0009-1 Qtolimod 1.5 mg/kg group for health status, deaths, mental state, behavioral activity, fecal characteristics, and other abnormalities showed no significant abnormalities.

Body weight changes of animals during the administration period are shown in Table 3 and Fig. 19. Before the first administration (at grouping), there was no significant difference in animal body weight between groups (P>0.05). On D3, D5, D9, and at the experimental endpoint (D11) after administration, compared to the saline control group, animal body weight in the T0009-1 Qtolimod 1.5 mg/kg group decreased significantly (D3: P<0.05, D5: P<0.05, D9: P<0.001, and endpoint: P<0.001).

**Table 3. Effect of T0009-1 Qtolimod on Body Weight of Animals in Mouse Melanoma B16-Luc Syngeneic Transplant Model**

| **Group** | **Numbe r** | **Body Weight (g)** | | | | | |
|---|---|---|---|---|---|---|---|
| | | **D1** | **D3** | **D5** | **D7** | **D9** | **Endpoint** |
| Control | 16 | 18.83±0.9 2 | 19.01±1.04 | 19.61±1.10 | 19.86±1.2 2 | 21.00±1.13 | 22.10±1.37 |
| T0009-1 Qtolimo d | 16 | 18.35±0.7 9 | 18.16±0.87 * | 18.87±0.77 * | 19.29±0.9 6 | 19.41±1.12** * | 19.64±0.93** * |

| | | | | | | | |
|---|---|---|---|---|---|---|---|
| * indicates P<0.05, *** indicates P<0.001 | | | | | | | |

Subcutaneous tumor volume changes during the administration period are shown in Table 4 and Fig. 20. Before the first administration (at grouping), there was no significant difference in animal subcutaneous tumor volume between groups (P>0.05). On D5, D7, D9, and at the experimental endpoint after administration, compared to the saline control group, animal subcutaneous tumor volume in the T0009-1 Qtolimod 1.5 mg/kg group decreased significantly (P<0.001).

**Table 4. Effect of T0009-1 Qtolimod on Subcutaneous Tumor Volume of Animals in Mouse Melanoma B16-Luc Syngeneic Transplant Model**

| **Group** | **Num ber** | **Subcutaneous Tumor Volume (mm³)** | | | | | |
|---|---|---|---|---|---|---|---|
| | | **D1** | **D3** | **D5** | **D7** | **D9** | **Endpoint** |
| Contro 1 | 16 | 83.17±1 1.60 | 158.27±4 4.25 | 355.09±122. 20 | 747.73±331.0 5 | 1431.52±550. 76 | 2136.54±779. 86 |
| T0009 -1 Qtolim od | 16 | 87.76±1 1.99 | 145.84±4 1.84 | 195.47±72.2 7*** | 220.45±106.2 3*** | 255.11±129.0 8*** | 325.79±172.2 3*** |

| | | | | | | | |
|---|---|---|---|---|---|---|---|
| *** indicates P<0.001 | | | | | | | |

Subcutaneous tumor weight changes are shown in 1-22. Compared to the saline control group, subcutaneous tumor weight in the T0009-1 Qtolimod 1.5 mg/kg group was significantly lower (***P<0.001), with a tumor inhibition rate of 90.43%.

Subcutaneous tumor weight was used to calculate the tumor inhibition rate using the formula: Tumor inhibition rate = (Negative control group tumor weight - Treatment group tumor weight) / Negative control group tumor weight × 100%. Relative tumor volume (RTV) was calculated as Vₜ/V₀, where V₀ is the tumor volume at grouping, and Vₜ is the tumor volume at each measurement. The anti-tumor efficacy of the compound was evaluated using T/C (%). Relative tumor proliferation rate T/C(%)= T_{RTV} / C_{RTV}*100%. (T_{RTV}: treatment group RTV; C_{RTV}: negative control group RTV). In principle, the evaluation criteria are: T/C (%) > 40% is ineffective; T/C (%) ≤ 40% and statistically significant P < 0.05 is effective.

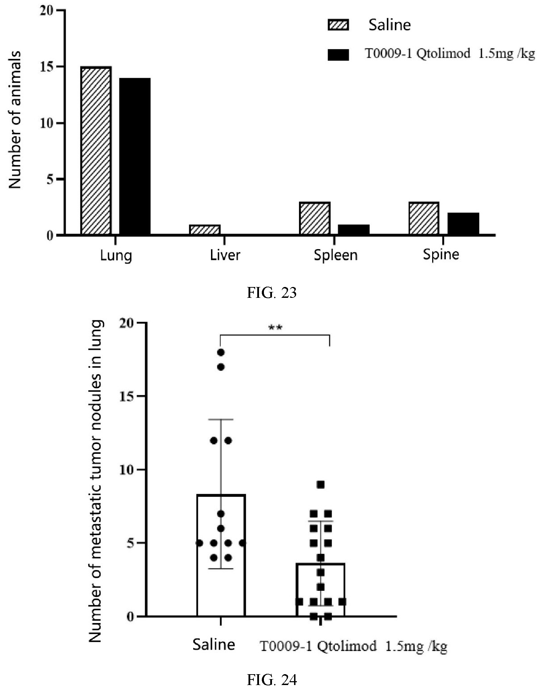

The effect of T0009-1 Qtolimod on tumor metastasis to various animal tissues is as follows. After intravenous syngeneic transplantation of mouse B16-Luc cells, visual inspection and counting revealed that the main sites of tumor metastasis were the lungs, also involving the spleen, kidneys, spine, femur or tibia, ovaries, abdominal cavity or abdominal fat, inner or outer abdominal wall, and intestines. The number of metastatic nodules in the lungs was high. After the above intratumoral injections, metastatic nodule counts were examined. Compared to the saline control group, the number of lung metastatic nodules in the T0009-1 Qtolimod 1.5 mg/kg group was significantly reduced (**P<0.01). Simultaneously, the total number of metastatic nodules in all tissues in the T0009-1 Qtolimod 1.5 mg/kg group was significantly reduced (**P<0.01). Results are shown in Figs. 23-25.

Under the experimental conditions, after 4 intratumoral injections administered once every 3 days, no deaths occurred in the T0009-1 Qtolimod 1.5 mg/kg group. Animal body weight decreased, which was likely due to the reduction in subcutaneous tumor size after analysis. T0009-1 Qtolimod 1.5 mg/kg significantly reduced the tumor volume and relative tumor volume of subcutaneous tumors in the mouse melanoma B16-Luc syngeneic transplant model, significantly lowered subcutaneous tumor weight, and showed good inhibitory effects on the subcutaneous tumors in the mouse melanoma B16-Luc syngeneic transplant model. Intratumoral injection of T0009-1 Qtolimod 1.5 mg/kg significantly reduced the number of metastatic lung nodules and the total number of metastatic nodules in all tissues in the mouse melanoma B16-Luc intravenous syngeneic transplant model, showing good inhibitory effects on systemic metastasis in the mouse melanoma B16-Luc syngeneic transplant model. In summary, under the experimental conditions, T0009-1 Qtolimod administered at 1.5 mg/kg via subcutaneous intratumoral injection once every 3 days for a total of 4 times (D1, D4, D7, and D10) showed good inhibitory effects on both subcutaneous tumors and systemic metastasis in the mouse melanoma B16-Luc syngeneic transplant model, while also extending animal survival. The dosing regimen for mice described above can be converted to a regimen for other animals, such as humans, based on technical knowledge in the field.

### Example 17. Efficacy of the Test Drug in Treating Mouse Head and Neck Cancer SCC-7 Subcutaneous Tumor Model

In this experiment, the method described in Examples 2-5 was used to encapsulate T0009-1 with lipid nanoparticles. Intratumoral administration was used to study its effect on tumor growth.

T0009-1 Qtolimod formulation was aliquoted in advance, stored at -20°C, and reconstituted on the day of use. SCC-7 cells (preserved by Hangzhou Moslite Biotechnology Co., Ltd.) were revived and cultured *in vitro* to obtain 5×10⁶ cells. Twenty-four 7-week-old female C3H mice were acclimated for one week, then weighed. The mouse head and neck cancer SCC-7 cell line was revived and passaged. When the cells were in good condition, SCC-7 cells (inoculum: 2e5) were transplanted subcutaneously into the right shoulder of the mice. Changes in mouse body weight and tumor size were recorded (the tumor-bearing mouse model was constructed by subcutaneous implantation of the SCC-7 cell line on the back). When the tumors reached approximately 80 mm³, the mice were grouped. Grouping and dosing information is shown in Table 5 below.

**Table 5. Mouse Grouping and Dosing**

| **Group** | **Compound Treatment** | **Number** | **Dose Volume (µl)** | **Administration Route** | **Dosing Frequency** |
|---|---|---|---|---|---|
| G1 | Saline (Vehicle) | 6 | 50 | Intratumoral (i.t.) | Once a week ×5 times (QW*5) |
| G2 | Qtolimod (T0009-1 Qtolimod) | 6 | 50 | i.t. | QW*5 |
| G3 | PD-1 mAb (Selleck, A2122) | 6 | 100 | Intraperitoneal (i.p.) | Twice a week ×6 times (BIW*6) |

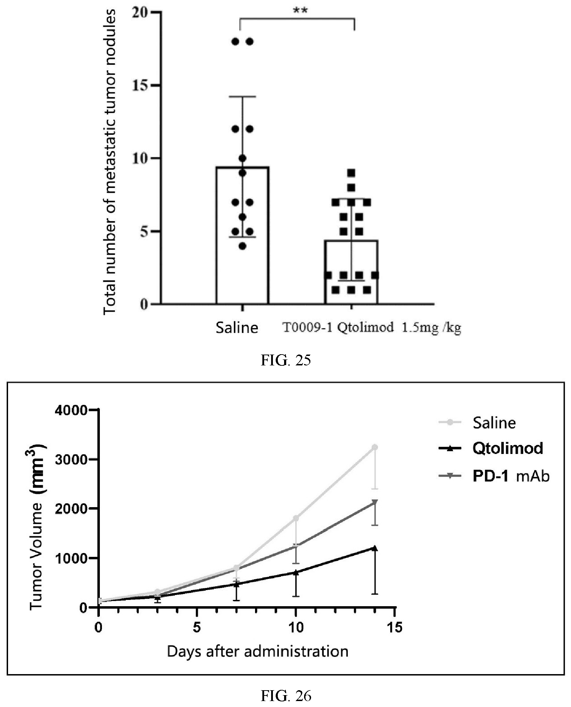

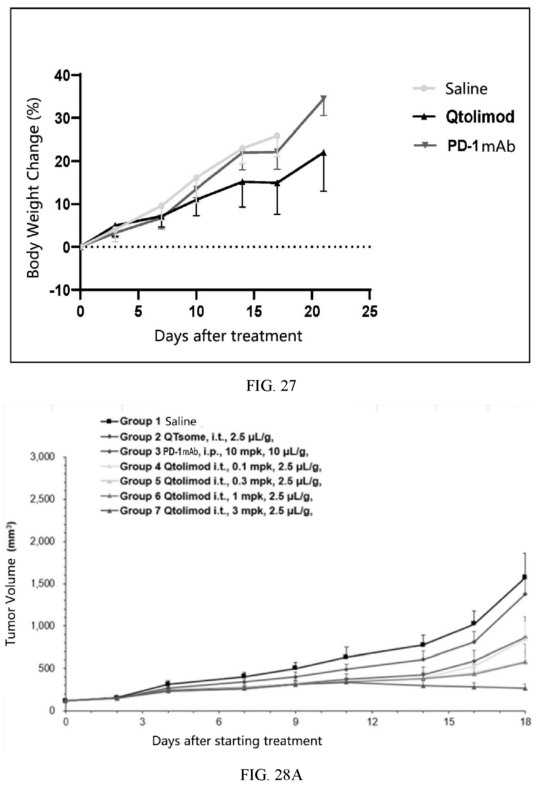

G1-G2 mice received intratumoral injections of 50 µL (administered at one or two sites as needed) at a dose of 1.5 mpk, for a total of 5 injections. The first injection day was Day 0. G3 positive control PD-1 mAb was administered subcutaneously at 100 µL, dose 10 mpk. The growth and proliferation of the back subcutaneous colon tumors in the experimental groups and control groups (G1 and G3) were observed and recorded daily, including measuring mouse body weight and tumor volume. The tumor growth inhibition rate (TGI) was calculated, and tumor growth curves were plotted (Figs. 26, 27). Under the experimental conditions, after intratumoral injection in the mouse head and neck cancer SCC-7 subcutaneous model, on Day 17, the positive control G3 PD-1 mAb group showed no significant tumor growth inhibition (TGI%=21.18%), while the G2 T0009-1 Qtolimod group significantly inhibited tumor growth (TGI%=57.28%). During the administration period, animal body weight decreased slightly, likely due to the reduction in subcutaneous tumor size after analysis.

### Example 18. Efficacy Study of the Test Drug in Balb/c Female Mouse EMT-6 and EMT-6-HPV18 Subcutaneous Transplant Models

This experiment used the method described in Examples 2-5 to prepare Qtolimod by encapsulating T0009-1 with lipid nanoparticles. Intratumoral administration was used to explore the efficacy of the test drug in Balb/c female mouse EMT-6 and EMT-6-HPV18 subcutaneous transplant models.

T0009-1 Qtolimod formulation was aliquoted in advance, stored at -20°C, and reconstituted on the day of use.

Mouse breast cancer cells EMT-6 and mouse breast cancer HPV-positive cells EMT-6-HPV18 (both preserved by Nantong WuXi AppTec Co., Ltd.) were cultured *in vitro* as monolayers. Culture conditions followed supplier technical instructions or references. Routine digestion and passaging with trypsin-EDTA were performed twice a week. When cell confluence reached 80%-90% and cell numbers met requirements, cells were harvested, counted, and inoculated. Tumor inoculation and grouping criteria: EMT-6 cells (5×10⁵ cells + Matrigel / 200 µL, PBS:Matrigel = 1:1) or EMT-6-HPV18 cells (5×10⁵ cells + Matrigel / 200 µL, PBS:Matrigel = 1:1) were subcutaneously inoculated into the right back of each mouse. When average tumor volume reached approximately 100 mm³, grouping and administration began. Grouping and dosing information is summarized in Table 6.

**Table 6. Mouse Grouping and Dosing**

| **Model** | **Group** | **Number** | **Compound Treatment** | **Dose (mg/kg)** | **Administration Route** | **Dosing Frequency** |
|---|---|---|---|---|---|---|
| EMT-6 | G1 | 6 | Saline (Vehicle) | - | i.t. | QW*4 |
| | G2 | 6 | QTsome | - | i.t. | QW*4 |
| | G3 | 6 | PD-1 mAb () | 10mpk | i.p. | BIW*6 |
| | G4 | 6 | Qtolimod | 0.1mpk | i.t. | QW*4 |
| | G5 | 6 | Qtolimod | 0.3mpk | i.t. | QW*4 |
| | G6 | 6 | Qtolimod | 1mpk | i.t. | QW*4 |
| | G7 | 6 | Qtolimod | 3mpk | i.t. | QW*4 |
| EMT-6-HPV18 | G8 | 6 | Saline | - | i.t. | QW*4 |
| | G9 | 6 | QTsome | - | i.t. | QW*4 |
| | G10 | 6 | PD-1 mAb () | 10mpk | i.p. | BIW*6 |
| | G11 | 6 | Qtolimod | 0.1mpk | i.t. | QW*4 |
| | G12 | 6 | Qtolimod | 0.3mpk | i.t. | QW*4 |
| | G13 | 6 | Qtolimod | 1mpk | i.t. | QW*4 |
| | G14 | 6 | Qtolimod | 3mpk | i.t. | QW*4 |

When subcutaneous tumor volume reached approximately 100 mm³, tumor-bearing mice with good tumor growth, no ulceration, and healthy status were selected and randomly grouped based on subcutaneous tumor volume (refer to Example 16). The grouping day was designated Day 0 (D0), and administration began according to animal body weight. The positive control PD-1 mAb was administered twice a week for a total of 6 times. Other drugs were administered once a week for a total of 4 times, at 2.5 ml/kg body weight, all via subcutaneous intratumoral injection. The negative control saline group received an equal volume of saline.

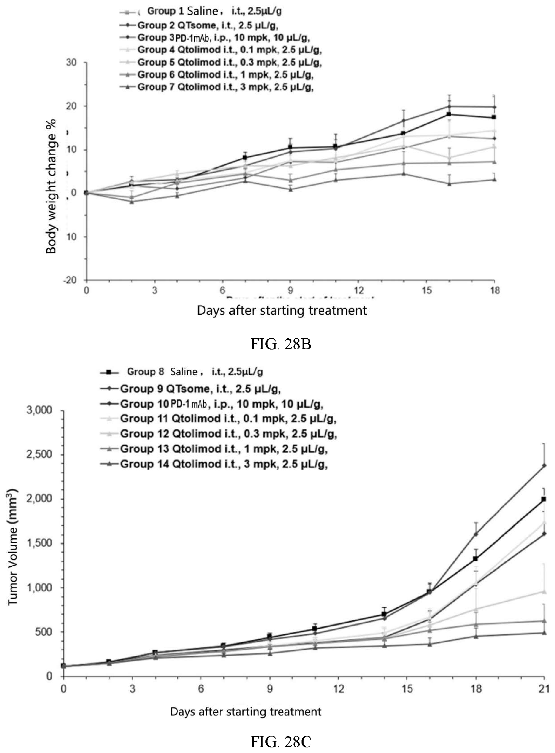

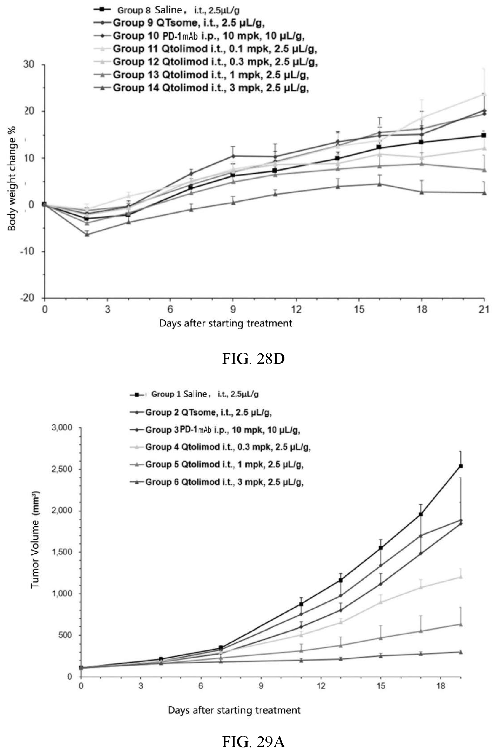

Experimental results are shown in Figs. 28A-D. Under the experimental conditions, after 4 intratumoral injections administered once a week for mouse breast cancer EMT6 (positive control PD-1 mAb was administered twice a week for 6 times), during the administration period, deaths occurred in the saline, QTsome, and PD-1 mAb groups: 2 deaths in the saline group, 1 death each in the QTsome and PD-1 mAb groups. In contrast, no deaths occurred in the Qtolimod 0.1 mg/kg, 0.3 mg/kg, 1 mg/kg, or 3 mg/kg groups. Animal body weight decreased during administration, likely due to the reduction in subcutaneous tumor size. Qtolimod 0.1 mg/kg (mpk) significantly reduced tumor volume and relative tumor volume of subcutaneous tumors in the mouse breast cancer EMT6 syngeneic transplant model, showing good inhibitory effects on subcutaneous tumors in the mouse breast cancer EMT6 syngeneic transplant model. On day 18 of administration, the tumor growth inhibition rates for Qtolimod 0.1, 0.3, 1, and 3 mg/kg against mouse breast cancer EMT6 were 49.9%, 67.9%, 68.4%, and 89.8%, respectively, all higher than the inhibition rate of PD-1 mAb. By day 38 of administration, all mice in the saline and QTsome groups had died, 2 mice survived in the positive control PD-1 mAb group, 2 survived in the Qtolimod 0.1 mpk group, 5 survived in the 0.3 mpk group, 5 survived in the 1 mpk group, and all survived in the 3 mpk group. Therefore, Qtolimod showed significant inhibitory effects on subcutaneous tumors in the mouse breast cancer EMT6 syngeneic transplant model and also extended animal survival.

This experiment also studied the EMT6-HPV18 mouse breast cancer model expressing the HPV18 antigen fragment. After 4 intratumoral injections administered once a week (positive control mPD-1 twice a week for 6 times), during the administration period, 1 mouse died in the QTsome group, while no deaths occurred in the saline, mPD-1, Qtolimod 0.1, 0.3, 1, or 3 mg/kg groups. Animal body weight decreased during administration, possibly due to the reduction in subcutaneous tumor size. Qtolimod 0.1 mg/kg significantly reduced tumor volume and relative tumor volume of subcutaneous tumors in the mouse breast cancer EMT6 syngeneic transplant model, showing good inhibitory effects on subcutaneous tumors in the mouse breast cancer EMT6 syngeneic transplant model. On day 21 of administration, the tumor growth inhibition rates for Qtolimod 0.3, 1, and 3 mg/kg against mouse breast cancer EMT6 were 55.1%, 75.2%, and 85.3%, respectively, all higher than the inhibition rate of PD-1 mAb. By day 38 of administration, all mice in the saline, QTsome, and Qtolimod 0.1 mpk groups had died, 1 mouse survived in the positive control PD-1 mAb group, 2 survived in the Qtolimod 0.3 mpk group, 4 survived in the 1 mpk group, and all survived in the 3 mpk group.

Comparing efficacy results between the EMT6 and EMT6-HPV18 mouse breast cancer syngeneic subcutaneous tumor models, it was found that both tumor cells expressing or not expressing HPV antigen showed significant tumor inhibition after Qtolimod treatment.

### Example 19. Efficacy of the Test Drug in Treating Balb/c Mouse Osteosarcoma K7M2 Subcutaneous Tumor Model

This experiment used the method described in Examples 2-5 to prepare Qtolimod by encapsulating T0009-1 with lipid nanoparticles. Intratumoral administration was used to explore the efficacy of the test drug in the Balb/c female mouse K7M2 subcutaneous transplant model.

The formulation was aliquoted in advance, stored at -20°C, and reconstituted on the day of use.

Mouse osteosarcoma cells K7M2 (preserved by Nantong WuXi AppTec Co., Ltd.) were cultured *in vitro* as monolayers. Culture conditions followed supplier technical instructions or references. Routine digestion and passaging with trypsin-EDTA were performed twice a week. When cell confluence reached 80%-90% and cell numbers met requirements, cells were harvested, counted, and inoculated. Tumor inoculation and grouping criteria: K7M2 cells (1×10⁶ cells) were subcutaneously inoculated into the right back of each mouse. When average tumor volume reached approximately 110 mm³, grouping and administration began. Grouping and dosing information is summarized in Table 7.

**Table 7. Mouse Grouping and Dosing**

| **Model** | **Group** | **Number** | **Compound Treatment** | **Dose (mg/kg)** | **Administration Route** | **Dosing Frequency** |
|---|---|---|---|---|---|---|
| | G1 | 5 | Saline (Vehicle) | - | i.t. | QW*3 |
| | G2 | 5 | QTsome | - | i.t. | QW*3 |
| K7M2 | G3 | 5 | PD-1 mAb () | 10mpk | i.p. | BIW*6 |
| | G4 | 5 | Qtolimod | 0.3mpk | i.t. | QW*3 |
| | G5 | 5 | Qtolimod | 1mpk | i.t. | QW*3 |
| | G6 | 5 | Qtolimod | 3mpk | i.t. | QW*3 |

When subcutaneous tumor volume reached approximately 110 mm³, tumor-bearing mice with good tumor growth, no ulceration, and healthy status were selected and randomly grouped based on subcutaneous tumor volume, and administration began according to animal body weight. The grouping day was designated Day 0. Drugs were administered once a week for a total of 3 times, while the positive control PD-1 mAb was administered twice a week for 6 times. Administration was at 2.5 mL/kg body weight, all via subcutaneous intratumoral injection. The negative control saline group received an equal volume of saline.

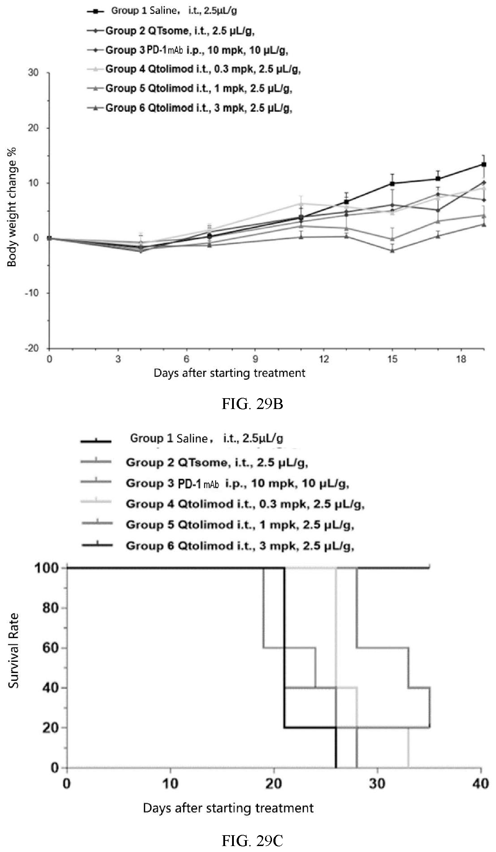

Results are shown in Figs. 29A-C. Under the experimental conditions, after 3 intratumoral injections administered once a week for mouse osteosarcoma K7M2 (positive control PD-1 mAb twice a week for 6 times), during the administration period, no deaths occurred in the Qtolimod 0.3, 1, or 3 mg/kg groups. Animal body weight decreased slightly during administration, possibly due to the reduction in subcutaneous tumor size. Qtolimod 0.1 mg/kg significantly reduced tumor volume and relative tumor volume of subcutaneous tumors in the mouse osteosarcoma K7M2 syngeneic transplant model, showing good inhibitory effects on subcutaneous tumors in the mouse osteosarcoma K7M2 syngeneic transplant model. On day 19 of administration, the tumor growth inhibition rates for Qtolimod 0.3, 1, and 3 mg/kg against mouse osteosarcoma K7M2 were 55.1%, 78.6%, and 92.2%, respectively, all higher than the inhibition rate of PD-1 mAb (TGI%=28.7%). By day 35 after grouping, all mice in the saline, QTsome, positive control mPD-1, and Qtolimod 0.3 mpk groups had died. In the 1 mpk group, 2 mice survived, and in the 3 mpk group, all mice survived with an average tumor volume of 539 mm³. Therefore, Qtolimod showed significant inhibitory effects on subcutaneous tumors in the mouse osteosarcoma K7M2 syngeneic transplant model and also extended animal survival.

### Example 20. Study on Drug Mechanism of Action at Different Time Points after Intratumoral Administration of the Test Drug in C57BL/6J Mouse Intestinal Cancer MC38 Subcutaneous Tumor Model

This experiment used the method described in Examples 2-5 to prepare Qtolimod by encapsulating T0009-1 with lipid nanoparticles. Intratumoral administration was used to study immune cell distribution at the administration site and in the spleen at different time points after administration.

The formulation was aliquoted in advance, stored at -20°C, and reconstituted on the day of use. MC38 cells were revived and cultured *in vitro* to obtain 5×10⁶ cells. Two hundred sixteen 7-week-old female C57BL/6 mice were acclimated for one week, then weighed. The mouse colon cancer MC38 cell line was revived and passaged. When the cells were in good condition, MC38 cells (inoculum: 1×10⁶) were transplanted subcutaneously into the right shoulder of the mice. Changes in mouse body weight and tumor size were recorded (the tumor-bearing mouse model was constructed by subcutaneous implantation of the MC38 cell line on the back). When tumor volume reached approximately 100 mm³, grouping and administration began. Grouping and dosing information is summarized in Table 8.

**Table 8. Mouse Grouping and Dosing**

| **Group** | **Number** | **Compound Treatment** | **Dose Volume (µl)** | **Administration Route** | **Dosing Frequency** |
|---|---|---|---|---|---|
| G1 | 54 | Vehicle (Saline) | 50 | i.t. | Every 3 days ×5 times (Q3D*5) |
| G2 | 54 | QTsome | 50 | i.t. | Q3D*5 |
| G3 | 54 | API (Free T0009-1) | 50 | i.t. | Q3D*5 |
| G4 | 54 | Qtolimod | 50 | i.t. | Q3D*5 |

Each mouse received an intratumoral injection of 50 µL (administered at one or two sites as needed) at a dose of 1.5 mpk, once every 3 days for a total of 5 injections. The first injection day was Day 0 (D0). After each administration, at 6h, 24h, and 48h, 3 mice (small, medium, and large tumors) from the group were euthanized. Tumor tissue and spleen samples were collected after death for RT-qPCR detection.
1. RNA Extraction: 100 mg of liver tissue was added to grinding beads and 500 µL Trizol lysis buffer, then ground in a homogenizer (parameters: 65 Hz/5 min). 100 µL of the homogenate was taken, 900 µL Trizol lysis buffer was added, and lysed for 10 min. 200 µL chloroform was added to each tube, thoroughly mixed, and placed on ice for 10 minutes to allow complete dissociation of nucleoprotein complexes. Centrifugation was performed at 4°C, 13000 rpm for 15 minutes. During this time, a new EP tube was prepared with 500 µL isopropanol pre-chilled on ice. After centrifugation, the upper aqueous phase (approximately 500 µL) was transferred to the new EP tube. It was left on ice for precipitation with equal volume of isopropanol for 10 min, followed by centrifugation at 13000 rpm for 10 minutes. Supernatant was removed, RNA precipitate was washed once with 1 mL 75% ethanol, centrifuged at 12000 rpm for 5 minutes. Supernatant was removed, RNA precipitate was air-dried or vacuum-dried for 5-10 minutes. RNA was dissolved in 30-50 µL DEPC-treated deionized water; the amount of water added for dissolution depended on the amount of RNA precipitate. Spectrophotometric analysis was then performed to determine sample concentration and purity.
2. Reverse Transcription: The following materials and steps were used to reverse transcribe RNA into cDNA. Genomic DNA removal: In an RNase-free centrifuge tube, the following mixture was prepared (reagents from Vazyme kit, R312-01): 2 µL 5×gDNA wiper Mix, RNase-free ddH2O to 10 µL, total RNA 1 µg. It was mixed gently by pipetting, and then incubated at 42°C for 2 min. Preparation of the first-strand cDNA synthesis reaction mixture: In an RNase-free centrifuge tube, the following mixture was prepared (reagents from Vazyme kit, R312-01): 10 µL of the mixture prepared in the previous step, 2 µL 10×RT Mix, 2 µL HiScript III Enzyme Mix, 1 µL Oligo(dT)20 VN, 1 µL Random hexamers, 4 µL RNase-free ddH2O. It was mixed gently by pipetting, and then incubated at 37°C for 15 min, 85°C for 5s to obtain first-strand cDNA. The reagent product was thawed and gently mixed: 2×qPCR Mix (abm, G891) and PCR Primers (Hangzhou Leqi, MQP026401, MQP103938, MQP076780, MQP101232, MQP093050, MQP091959, MQP031788, MQP093658, MQP072830, MQP025979, MQP091399, MQP029630, MQP028834, MQP025254, MQP077323, MQP076358, MQP092655, MQP027158). The qPCR reaction system on ice was prepared according to Table 9 below.

**Table 9. qPCR Reaction System**

| **Material** | **Volume** |
|---|---|
| **2×qPCR Mix** | 10µL |
| **First-strand cDNA** | 2µL |
| **PCR Primer(2µM)** | 2µL |
| **RNase-free ddH2O** | 6µL |

The qPCR reaction system was gently mixed and briefly centrifuged. The three-step qPCR program was set up according to Table 10 or a Roche L96 qPCR instrument was used to set the program. Data processing: 2^{-ΔΔCt}.

**Table 10. Three-Step qPCR Program**

| **Cycle** | **Step** | **Time** | **Detection?** |
|---|---|---|---|
| **1** | Pre-denaturation | 10min | No |
| **40** | Denaturation | 10 sec | No |
| | Annealing | 20 sec | No |
| | Extension | 15 sec | Yes |
| **1** | Melt Curve Analysis | Heating rate 0.5°C/6 sec | Yes |
| | Cooling | 30 sec | No |

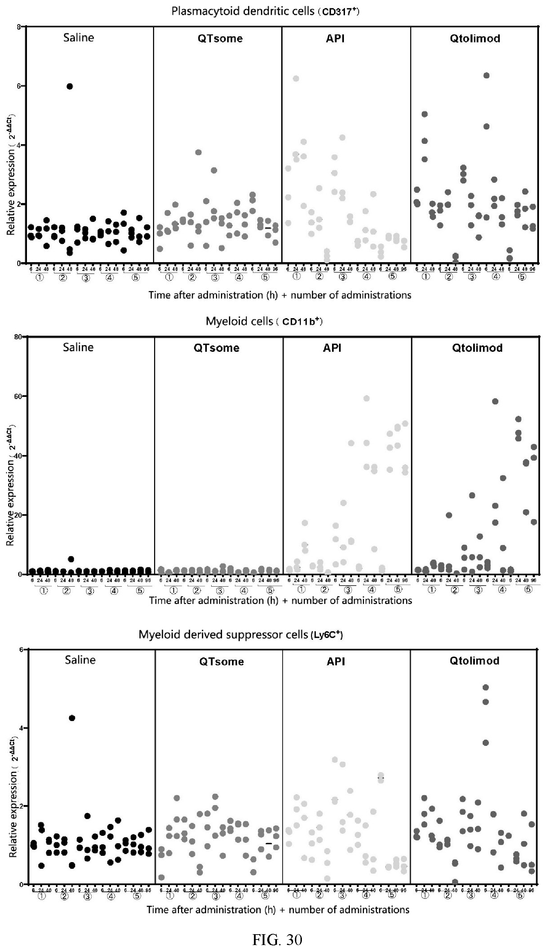

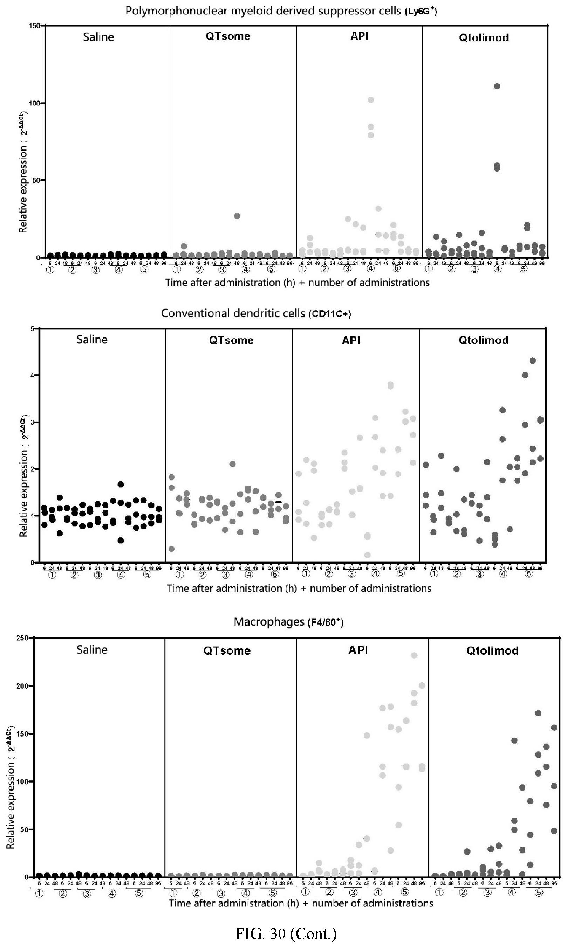

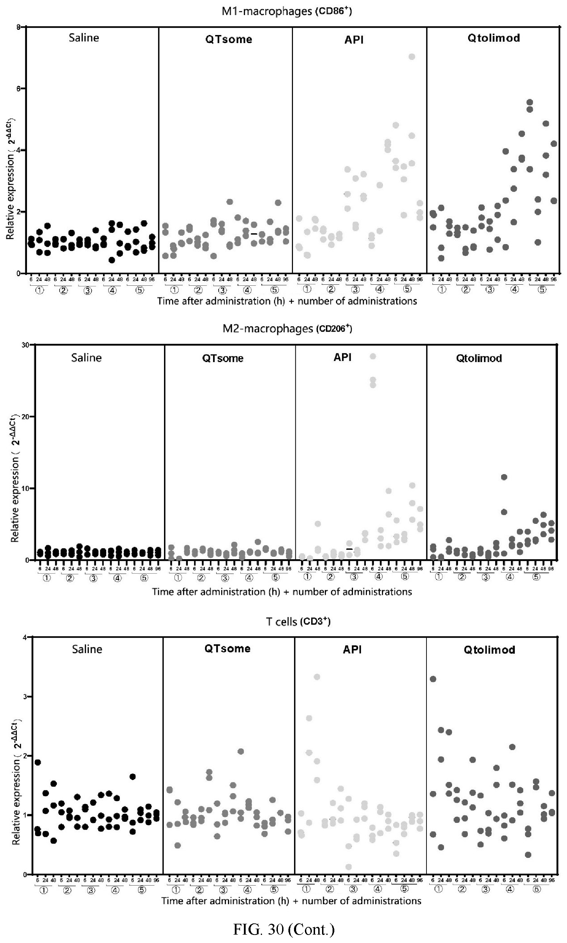

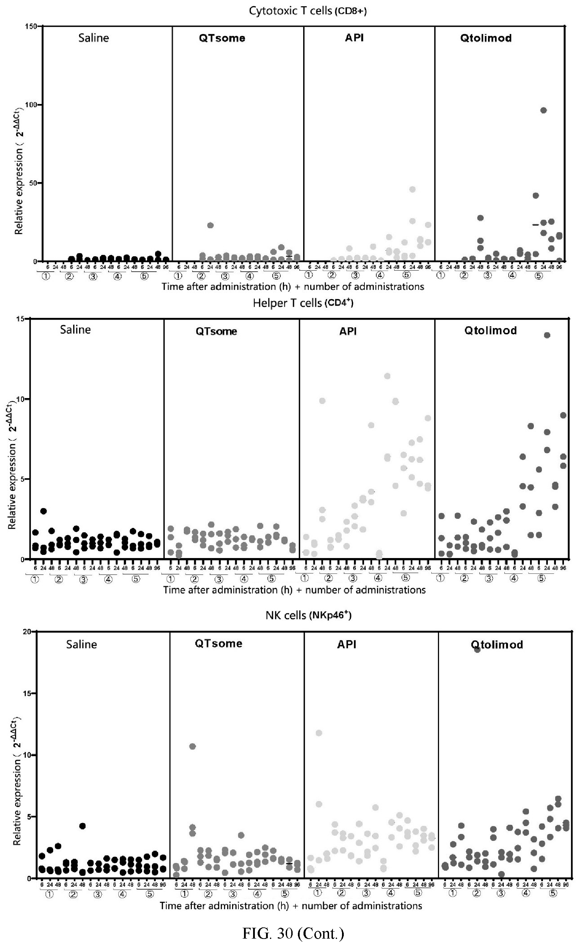

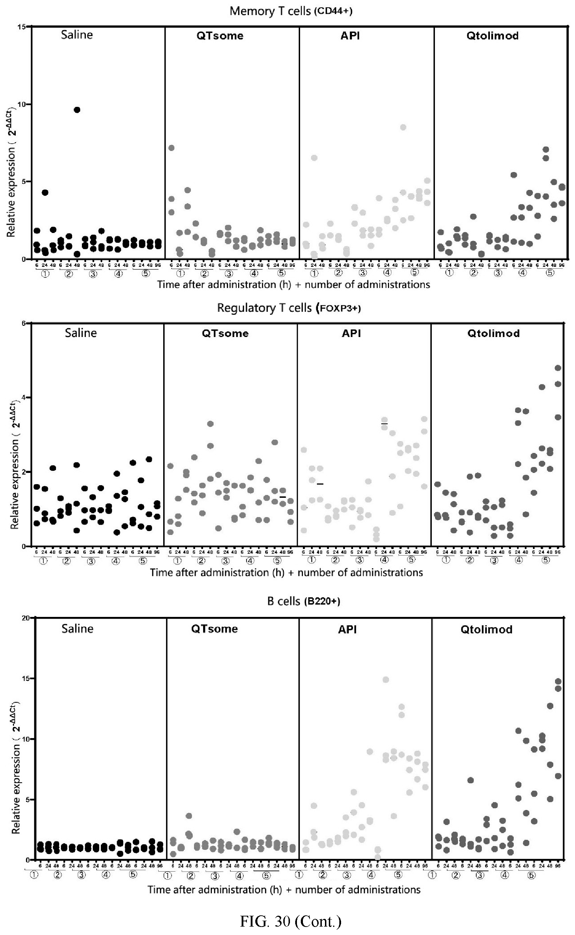

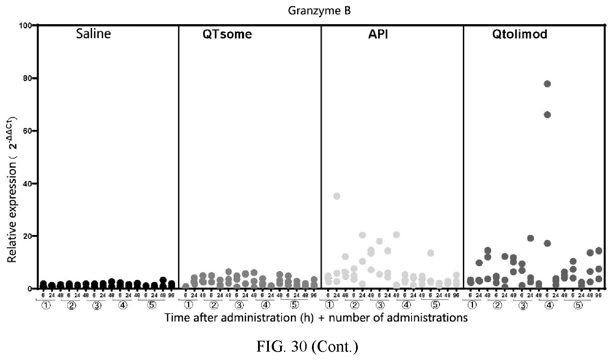

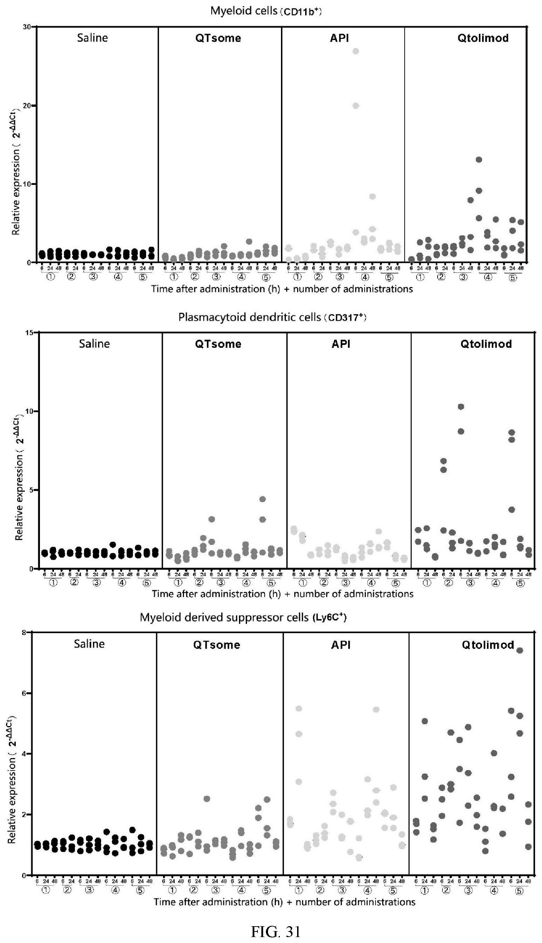

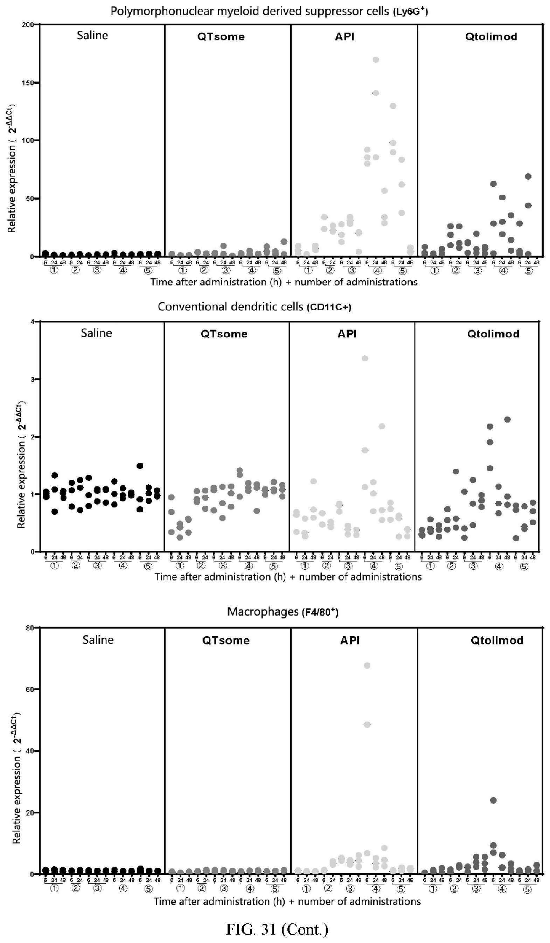

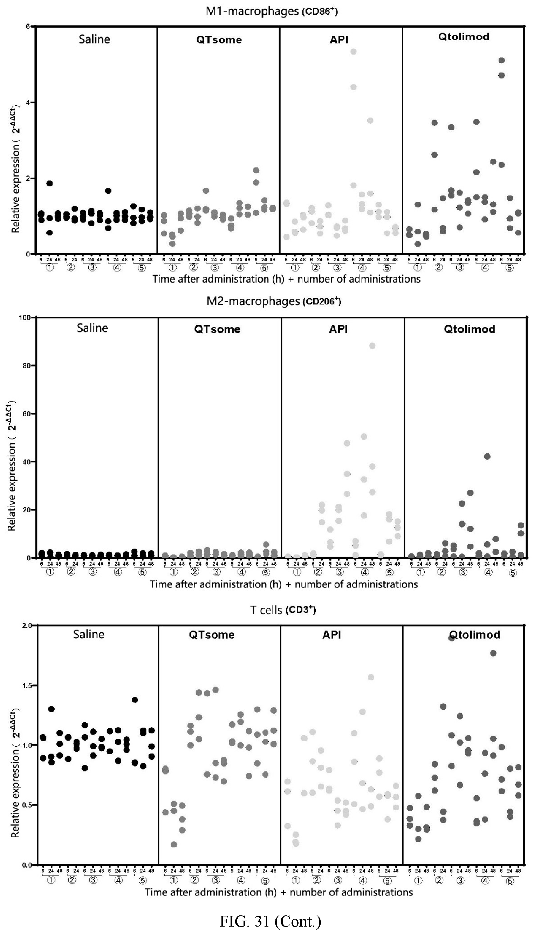

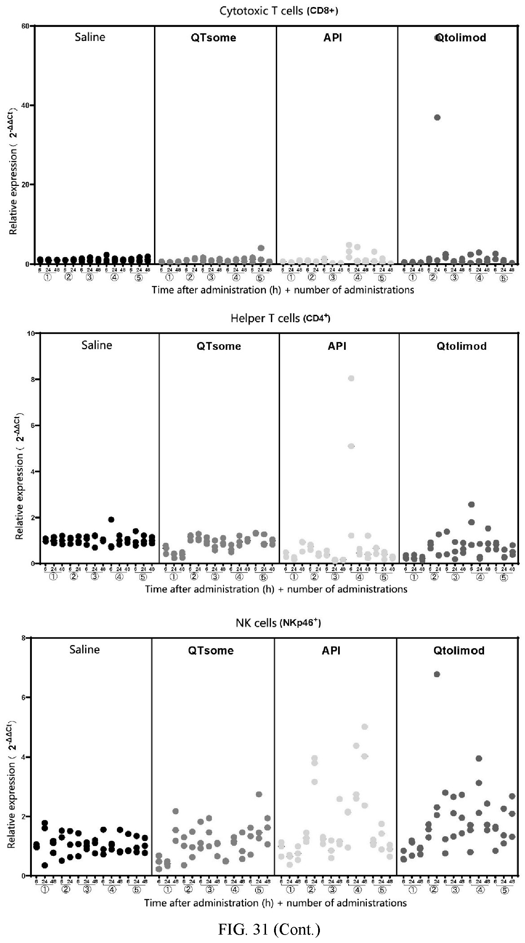

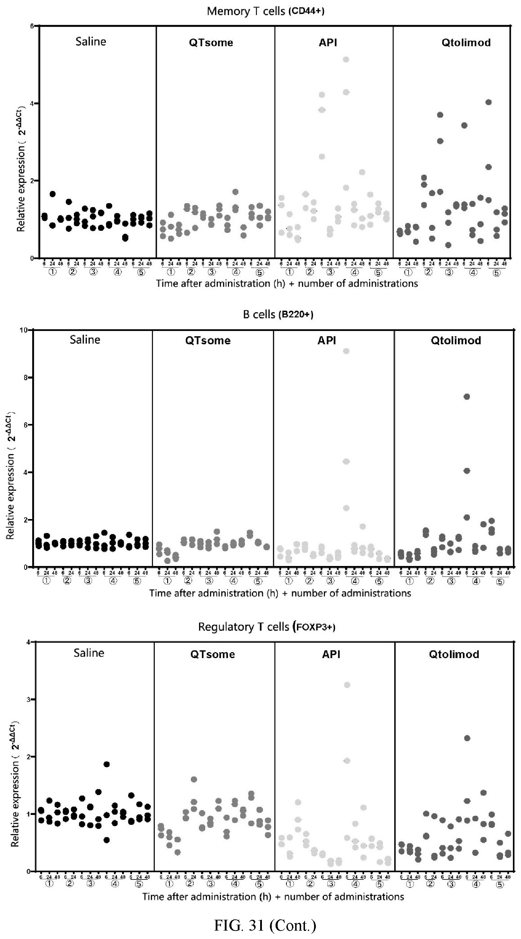

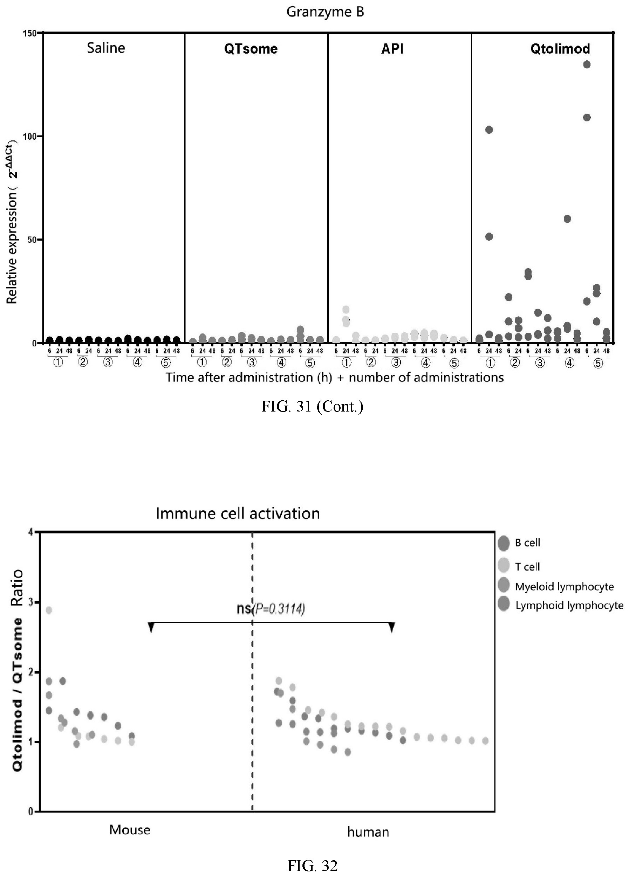

Mice received intratumoral injections of 1.5 mpk, Q3D*5. After the first four administrations, samples were taken at 6h, 24h, and 48h post-administration. After the fifth administration, samples were taken at 6h, 24h, 48h, and 96h post-administration. Mice were euthanized, and tumor and spleen tissues were collected for RT-qPCR detection of multiple immune cell markers to analyze immune cell activation after administration. Results are shown in Figs. 30-31. Monocytes/macrophages in tumor tissue were significantly upregulated after administration (approximately 60-fold), and upregulation became more pronounced with increasing number of administrations. Macrophages are widely involved in immune responses, immune effects, and immune regulation, playing key roles in cancer therapy. Activated macrophages can effectively exert anti-tumor effects, phagocytose tumor cells, secrete cytotoxic cytokines to directly or indirectly exert anti-tumor effects, produce certain enzymes and reactive oxygen species to directly kill or inhibit tumor cell growth, etc. Simultaneously, detection results from the spleen showed that monocytes/macrophages were not significantly upregulated after administration, indicating that monocyte/macrophage activation was concentrated at the administration site. Qtolimod activated plasmacytoid dendritic cells (pDCs). Activated pDCs recruit large numbers of T cells into the tumor microenvironment by producing large amounts of IFN-α and other cytokines, thereby exerting tumor-killing effects. Experimental results found that intratumoral administration significantly upregulated plasmacytoid dendritic cells (pDCs), while also significantly activating immune cells such as macrophages, CD3+ T cells, CD8+ T cells, and NK cells, and releasing granzyme B (GZMB), promoting the tumor cell killing process. Meanwhile, macrophages phagocytose tumor cell fragments, promoting tumor antigen presentation and anti-tumor cellular immune activation. In contrast, various immune cells in the spleen remained unactivated or mildly activated after administration, indicating that Qtolimod concentratedly induces anti-tumor immune responses locally at the tumor site, with a lower risk of causing systemic side effects.

### Example 21. Study on Immune Activation after In Vitro Stimulation of Mouse and Human PBMC Cells with the Test Drug

This experiment used the method described in Examples 2-5 to prepare Qtolimod by encapsulating T0009-1 with lipid nanoparticles. The prepared Qtolimod was used to stimulate PBMC cells *in vitro.* Cells were collected 24h later for proteomic sequencing analysis. Qtolimod formulation was aliquoted in advance, stored at -20°C, and reconstituted on the day of use.

Human PBMC cells were purchased from Maishun Biotechnology Co., Ltd in Zhejiang Free Trade Zone. Mouse PBMC cell isolation steps: A 10 ml sterile siliconized centrifuge tube was prepared, 4 ml separation medium was added, then 0.5-2.0 ml blood sample was slowly layered on top. The blood sample was carefully layered onto the separation medium interface. Centrifugation was performed at 450g for 30 min. After centrifugation, the ring-shaped milky white mononuclear cell layer was carefully aspirated from the centrifuge tube and transferred to a new centrifuge tube. 10 ml wash buffer was added to the tube containing mononuclear cells, cells were mixed. Centrifugation was performed at 250g for 10 min. Supernatant was discarded. Cells were resuspended in 5 ml PBS. Centrifugation was performed at 250g for 10 min, and supernatant was discarded. Washing was repeated twice, supernatant was discarded, and cells were resuspended in 0.5 mL RPMI-1640 culture medium. Mouse and human PBMC cells were plated in 6-well plates at 1×10⁶/well, and stimulated with 5 µg Qtolimod. Cells were collected 24h later and sent to Jingjie Biotechnology for 4D-Smart DIA proteomic sequencing analysis. Results are shown in Fif. 32. The test drug stimulated human and mouse PBMC cells *in vitro.* Cells were collected 24h later for protein sequencing analysis. Results showed that compared to the saline (Vehicle) group, Qtolimod activated immune cells to a certain extent, including B cell pathway genes, T cell pathway genes, myeloid lymphocyte pathway genes, and lymphoid lymphocyte pathway genes, and there was no significant difference in the degree of activation between human and mouse immune cells.

B cell pathway representative genes (mouse: Beap29, Cd72, Blnk, Bcl11a, Bel7b, Bcap31, Cd22) (human: BCL3, CD72, BCAP31, BCAP29, CD22, CD79B, BCL9L, BANK1, BCL10, PBXIP1). T cell pathway representative genes (mouse: Tgtp2, Cd2, Itfg1, Trac, Nfatc1, Art2b, Cd247) (human: LAT2, CD8B2, SART3, CD247, LAT, CD7, VSIR, CD3E, NFATC2, NFATC3, CD5, CD3D, CD4, CD6, CD3G, CD2). Myeloid lymphocyte pathway representative genes (mouse: Mndal, Mlf2, Myd88) (human: MYADM, MNDA, CD33, MLF2, MYD88, MYDGF). Lymphoid lymphocyte pathway genes (mouse: Ly6d, Lsp1, Ly9, Cd19) (human: LCP2, CD19, BTLA, LSP1, LY75).

### Example 22. Efficacy Study of the Test Drug in an Orthotopic Bladder Cancer (MB49) Model

This experiment used the method described in Examples 2-5 to prepare Qtolimod by encapsulating T0009-1 with lipid nanoparticles. An orthotopic bladder cancer mouse model was established by transplanting mouse bladder cancer MB49 cells into the bladder mucosa. The therapeutic effect of Qtolimod on the orthotopic syngeneic transplant model of bladder cancer MB49 was evaluated via intravesical instillation.

Qtolimod formulation was aliquoted in advance, stored at -20°C, and reconstituted on the day of use. Mouse MB49-Luc cells were cultured *in vitro* under conditions: high-glucose DMEM medium, 37°C, 5% CO2. Routine medium changes and digestion/passaging were performed 1-2 times per week. When cell numbers met requirements, logarithmically growing cells were harvested, counted, and inoculated. Mouse bladder cancer MB49-Luc cells in logarithmic growth phase were digested with 0.5% trypsin, resuspended in sterile PBS buffer to 4×10⁷/ml, and 50 µl/mouse was injected into the bladder mucosa of C57BL/6J mice to construct the orthotopic transplant tumor model. Five days post-modeling, all mice were injected with the substrate potassium luciferin and underwent *in vivo* imaging. Tumor-bearing mice with healthy growth were selected for grouping, randomly grouped based on fluorescence intensity values (details in Table 9), ensuring intra-group tumor fluorescence intensity variation did not exceed 10-fold. Combined with palpation, animals with abnormally large orthotopic tumors were excluded. Administration began the next day (day 6 post-modeling). The administration day was designated Day 1 (D1), and administration started at 50 µL/mouse, once every 7 days for a total of 3 times (D1, D8, D15), all via intravesical instillation. The saline control group received an equal volume of saline.

For tumor monitoring, all animals were monitored before grouping, and grouped animals were monitored after grouping. Tumor status was monitored at grouping (before first administration), every 7 days after grouping, and before euthanasia (D1, D7, D14, D21) via *in vivo* imaging to monitor fluorescence intensity of orthotopic bladder cancer, comparing differences in orthotopic tumor growth changes between groups. Simultaneously, orthotopic tumor size grade was determined by palpation twice weekly and before euthanasia (D1, D5, D8, D12, D15, D19, D22, D35). Tumors were graded by size into 4 levels: Level 0, almost no palpable mass; Level 1, palpable very small mass (estimated tumor diameter within 2 mm); Level 2, palpable moderate-sized mass (estimated diameter 2-5 mm); Level 3, palpable large mass (estimated diameter 5-10 mm); Level 4, palpable very large mass (estimated diameter over 10 mm). By comparing tumor size grades via palpation, differences in orthotopic tumor size between groups were compared.

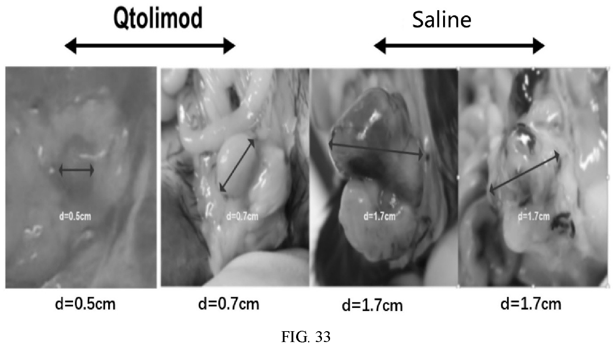

Results are shown in Fig. 33. Under the experimental conditions, intravesical instillation was administered once a week for 3 times. Due to rapid growth of the orthotopic transplanted tumor, the fluorescence substrate could not enter the tumor interior in later stages, so palpation was used for observation during the observation period. At day 35 during administration, anatomical observation was consistent with palpation findings. In the saline control group, tumor diameter reached 1.7 cm, oval shape, tumor growth caused bladder distension, with necrosis and congestion inside the tumor. Simultaneously, metastatic tumors appeared in the kidneys and large intestine. In the Qtolimod 4 mpk treatment group, tumor diameters were 0.5 cm and 0.7 cm, with rounder morphology, and no metastases were found. This indicates that the test drug significantly inhibited tumor growth and alleviated behavioral impacts in mice caused by bladder tumor growth.

The above describes only some embodiments of the present disclosure. For those of ordinary skill in the art, various modifications and improvements can be made without departing from the creative concept of the present disclosure, and these all fall within the scope of protection of the present disclosure.

## Claims

1. A Toll-like receptor agonist, **characterized by** comprising any one of the following (a)-(c):
(a) a modified or unmodified nucleotide set forth in any one of SEQ ID NOs: 1-4;
(b) a functional variant of a modified or unmodified nucleotide set forth in any one of SEQ ID NOs: 1-4;
(c) a pharmaceutically acceptable salt of the nucleotide of (a) or (b);
wherein the nucleotide is entirely or partially composed of ribonucleotides or entirely or partially composed of deoxyribonucleotides.

2. The Toll-like receptor agonist according to claim 1, **characterized in that** the modification comprises one or more of the following: replacing the P=O bond between all or part of adjacent two nucleotides in the sequence with a P=S bond; replacing all or part of the deoxyribonucleotides in the sequence with ribonucleotides, or replacing all or part of the ribonucleotides in the sequence with deoxyribonucleotides; replacing the 2'-OH group of ribonucleotides or deoxyribonucleotides with methoxy, methoxyethyl, or halogen; bridging the 2nd and 4th carbon atoms of the nucleotide to form locked nucleic acid or (S)-cEt-BNA.

3. An active ingredient encapsulated by a lipid component, **characterized in that** the active ingredient comprises the Toll-like receptor agonist according to claim 1 or 2.

4. The active ingredient encapsulated by a lipid component according to claim 3, **characterized in that** the mass ratio of the lipid component to the Toll-like receptor agonist according to claim 1 or 2 is (8-12):1.

5. The active ingredient encapsulated by a lipid component according to claim 3, **characterized in that** the lipid component comprises one or more of the following: cationic polymers, lipid nanoparticles (LNP), cationic liposomes, QTsome, lipopolyplexes, microparticles, microspheres, and nanoemulsions.

6. The active ingredient encapsulated by a lipid component according to claim 3, **characterized in that** the lipid component comprises one or more of cationic lipids, ionizable lipids, neutral lipids, and PEGylated lipids.

7. The active ingredient encapsulated by a lipid component according to claim 6, **characterized in that** the lipid component comprises DOTAP, DODMA, DSPC, cholesterol, and DMG-PEG, and the molar ratio of DOTAP:DODMA:DSPC:cholesterol:DMG-PEG is (1-30):(5-50):(5-40):(15-60):(0-10); preferably, the lipid component comprises DOTAP, DODMA, DSPC, cholesterol, and DMG-PEG, and the molar ratio of DOTAP: DODMA: D SPC: cholesterol: DMG-PEG is 15:25:20:38.5:1.5.

8. The active ingredient encapsulated by a lipid component according to claim 3 or 7, **characterized in that** the active ingredient further comprises an additional therapeutic agent, preferably, the molar ratio of the additional therapeutic agent to the Toll-like receptor agonist according to claim 1 or 2 is (0.1-5): 1, preferably 1:2.

9. The active ingredient encapsulated by a lipid component according to claim 8, **characterized in that** the additional therapeutic agent is one or more agents selected from the group consisting of antibodies, chemotherapeutic drugs, and small molecule drugs; preferably, is at least one selected from the group consisting of pattern recognition receptor agonists other than the Toll-like receptor agonist according to claim 1 or 2, antibodies, anthracyclines, camptothecins, immunogenic cell death inducers, tyrosine kinase inhibitors, and taxanes; more preferably, the additional therapeutic agent is one or both of resiquimod and doxorubicin.

10. A method for preparing the active ingredient encapsulated by a lipid component according to claim 3, **characterized by** comprising the following steps (a)-(f):
(a) preparing the Toll-like receptor agonist according to claim 1 or 2 as an acidic solution;
(b) preparing each component of the lipid components as a lipid ethanol solution;
(c) mixing and reacting the solutions prepared in steps (a) and (b) to form an initial lipid nanoparticle solution;
(d) optionally repeating steps (a)-(c) to obtain more initial lipid nanoparticle solutions;
(e) removing ethanol from the initial lipid nanoparticle solution, adjusting the active ingredient concentration and pH to obtain a lipid nanoparticle suspension;
(f) optionally mixing an additional therapeutic agent with the lipid nanoparticle suspension to prepare a multi-active-ingredient liposome.

11. A composition, **characterized by** comprising the active ingredient encapsulated by a lipid component according to any one of claims 3-7 and an additional therapeutic agent, preferably, the additional therapeutic agent is one or both of resiquimod and doxorubicin.

12. A delivery system, **characterized by** comprising an active ingredient and a lipid component, wherein the active ingredient comprises the Toll-like receptor agonist according to claim 1 or 2.

13. The delivery system according to claim 12, **characterized in that** the mass ratio of the lipid component to the Toll-like receptor agonist according to claim 1 or 2 is (8-12):1, preferably 10:1.

14. The delivery system according to claim 12, **characterized in that** the lipid component comprises one or more of cationic lipids, ionizable lipids, neutral lipids, and PEGylated lipids; preferably, the lipid component comprises DOTAP, DODMA, DSPC, cholesterol, and DMG-PEG, and the molar ratio of DOTAP:DODMA:DSPC:cholesterol:DMG-PEG is (1-30):(5-50):(5-40):(15-60):(0-10); more preferably, the lipid component comprises DOTAP, DODMA, DSPC, cholesterol, and DMG-PEG, and the molar ratio of DOTAP:DODMA:DSPC:cholesterol:DMG-PEG is 15:25:20:38.5:1.5.

15. The delivery system according to any one of claims 12-14, **characterized in that** the active ingredient further comprises an additional therapeutic agent, preferably, the additional therapeutic agent is one or both of resiquimod and doxorubicin.

16. A method for preparing the delivery system according to claim 12, **characterized by** comprising the following steps (a)-(d):
(a) preparing a Toll-like receptor agonist buffer solution using a buffer and the Toll-like receptor agonist according to claim 1 or 2;
(b) preparing a lipid ethanol solution using anhydrous ethanol and the lipid component;
(c) mixing the lipid ethanol solution and the Toll-like receptor agonist buffer solution at a mass ratio of lipid component to Toll-like receptor agonist of (8-12):1;
(d) removing ethanol, diluting, and adjusting pH to 6.8-7.2.

17. A pharmaceutical composition, **characterized by** comprising the Toll-like receptor agonist according to claim 1 or 2, the active ingredient encapsulated by a lipid component according to any one of claims 3-9, the composition according to claim 11, or the delivery system according to any one of claims 12-15, and a pharmaceutically acceptable carrier or excipient.

18. A vaccine adjuvant or a vaccine, **characterized by** comprising the Toll-like receptor agonist according to claim 1 or 2, the active ingredient encapsulated by a lipid component according to any one of claims 3-9, the composition according to claim 11, the delivery system according to any one of claims 12-15, or the pharmaceutical composition according to claim 17.

19. Use of the Toll-like receptor agonist according to claim 1 or 2, the active ingredient encapsulated by a lipid component according to any one of claims 3-9, the composition according to claim 11, the delivery system according to any one of claims 12-15, or the pharmaceutical composition according to claim 17 in any one of the following (a)-(j):
(a) use in the manufacture of a medicament for preventing tumorigenesis;
(b) use in the manufacture of a medicament for treating a tumor;
(c) use in the manufacture of a medicament for inhibiting tumor growth;
(d) use in the manufacture of a medicament for preventing tumor cell metastasis;
(e) use in the manufacture of a medicament for preventing tumor recurrence;
(f) use in the manufacture of a vaccine;
(g) use in the manufacture of a medicament for treating or preventing an inflammation-related disease or an infectious disease;
(h) use in the manufacture of a medicament for immune activation;
(i) use in the manufacture of a medicament for upregulating cytokine expression;
(j) use for upregulating cytokine expression for non-therapeutic purpose.

20. The use according to claim 19, **characterized in that** the immune activation comprises B cell pathway activation, T cell pathway activation, myeloid lymphocyte pathway activation, lymphoid lymphocyte pathway activation, macrophage activation, plasmacytoid dendritic cell (pDC) activation, B cell activation, T cell activation, CD3+ T cell activation, CD8+ T cell activation, NK cell activation, and granulocyte activation.

21. The use according to claim 19, **characterized in that** the tumor is selected from solid tumors or tumors into which drugs can be injected, such as at least one of head and neck cancer, esophageal cancer, oral cancer, nasopharyngeal cancer, thyroid cancer, lung cancer, gastric cancer, liver cancer, pancreatic cancer, spleen cancer, non-small cell lung cancer, colorectal cancer, colon cancer, intestinal cancer, kidney cancer, brain tumor, glioma, bladder cancer, prostate cancer, breast cancer, ovarian cancer, fallopian tube cancer, cervical cancer, uterine cancer, bone cancer, osteosarcoma, chondroma, liposarcoma, neuroblastoma, synovial sarcoma, astrocytoma, glioblastoma multiforme, anaplastic astrocytoma, peritoneal cavity cancer, soft tissue sarcoma, sarcoma, rhabdomyosarcoma, advanced myxoid disease, melanoma, and skin cancer.

22. The use according to claim 19, **characterized in that** the medicament is administered via one or more of the following routes: intratumoral injection, intravenous injection, subcutaneous injection, intramuscular injection, intra-arterial injection, intraperitoneal injection, intra-central-nervous-system injection, intravesical instillation, interventional therapy, oral, transdermal, pulmonary, ocular, and topical administration.
